# EUROPEAN PATENT APPLICATION

(11) **EP 2 336 341 A1**
(43) Date of publication of application: **22.06.2011**
(21) Application number: 09180143.1
(22) Date of filing: 21.12.2009
(51) Int. Cl.: C12P 5/00, C12P 5/02, C12N 9/88

(54) **Method for producing an alkene comprising the step of converting an alcohol by an enzymatic dehydration step**

(71) Applicant: Marliere, Philippe, 75020 Paris (FR)
(72) Inventor: Marliere, Philippe, 75020 Paris (FR)
(74) Representative: Heunemann, Dieter

(57) **Abstract**

Described is a method for producing an alkene comprising the step of converting an alcohol by an enzymatic dehydration step. The conversion preferably makes use of an unsaturated fatty acid hydratase, in particular of an oleate hydratase (EC 4.2.1.53). Moreover, the present invention describes a recombinant organism which produces an alcohol and which expresses a hydratase enzyme which is capable of catalyzing the conversion of said alcohol into an alkene.

## Description

The present invention relates to a method for producing an alkene comprising the step of converting an alcohol by an enzymatic dehydration step. The conversion preferably makes use of an unsaturated fatty acid hydratase, in particular of an oleate hydratase (EC 4.2.1.53). Moreover, the present invention relates to a recombinant organism which produces an alcohol and which expresses a hydratase enzyme which is capable of catalyzing the conversion of said alcohol into an alkene.

A large number of chemical compounds are currently derived from petrochemicals. Alkenes (such as ethylene, propylene, the different butenes, or else the pentenes, for example) are used in the plastics industry, for example for producing polypropylene or polyethylene, and in other areas of the chemical industry and that of fuels. Ethylene, the simplest alkene, lies at the heart of industrial organic chemistry: it is the most widely produced organic compound in the world. It is used in particular to produce polyethylene, a major plastic. Ethylene can also be converted to many industrially useful products by reaction (e.g. by oxidation or halogenation). Propylene plays a similarly important role: its polymerization results in a plastic material, polypropylene. The technical properties of this product in terms of resistance, density, solidity, deformability, and transparency are unequalled. The worldwide market for polypropylene has grown continuously since its invention in 1954. Butylene exists in four forms, one of which, isobutylene, enters into the composition of methyl-tert-butyl-ether (MTBE), an anti-knock additive for automobile fuel. Isobutylene can also be used to produce isooctene, which in turn can be reduced to isooctane (2,2,4-trimethylpentane); the very high octane rating of isooctane makes it the best fuel for so-called "gasoline" engines. Amylene, hexene and heptene exist in many forms according to the position and configuration of the double bond. These products have real industrial applications but are less important than ethylene, propylene or butenes. All these alkenes are currently produced by catalytic cracking of petroleum products (or by a derivative of the Fischer-Tropsch process in the case of hexene, from coal or gas). Their production costs are therefore tightly linked to the price of oil. Moreover, catalytic cracking is sometimes associated with considerable technical difficulties which increase process complexity and production costs.

The production by a biological pathway of alkenes or other organic molecules that can be used as fuels or as precursors of synthetic resins is called for in the context of a sustainable industrial operation in harmony with geochemical cycles. The first generation of biofuels consisted in the fermentative production of ethanol, as fermentation and distillation processes already existed in the food processing industry. The production of second generation biofuels is in an exploratory phase, encompassing in particular the production of long chain alcohols (butanol and pentanol), terpenes, linear alkanes and fatty acids. Two recent reviews provide a general overview of research in this field: Ladygina et al. (Process Biochemistry 41 (2006),1001) and Wackett (Current Opinions in Chemical Biology 21 (2008), 187).

The production of ethylene by plants has long been known (Meigh et al. (Nature 186 (1960), 902)). According to the metabolic pathway elucidated, methionine is the precursor of ethylene (Adams and Yang (PNAS 76 (1979), 170)). Conversion of 2-oxoglutarate has also been described (Ladygina et al. (Process Biochemistry 41 (2006), 1001). Since a single ethylene molecule requires the previous production of a four- or five-carbon chain, the equipment and energy needs of all these pathways are unfavorable and do not bode well for their industrial application for alkene bioproduction.
Moreover, many microorganisms are capable of producing propylene, however, with an extremely low yield
The conversion of isovalerate to isobutylene by the yeast *Rhodotorula minuta* has been described (Fujii et al. (Appl. Environ. Microbiol. 54 (1988), 583)), but the efficiency of this reaction, less than 1 millionth per minute, or about 1 for 1000 per day, is far from permitting an industrial application. The reaction mechanism was elucidated by Fukuda et al. (BBRC 201 (1994), 516) and involves a cytochrome P450 enzyme which decarboxylates isovalerate by reduction of an oxoferryl group Fe^{v}=O. Large-scale biosynthesis of isobutylene by this pathway seems highly unfavorable, since it would require the synthesis and degradation of one molecule of leucine to form one molecule of isobutylene. Also, the enzyme catalyzing the reaction uses heme as cofactor, poorly lending itself to recombinant expression in bacteria and to improvement of enzyme parameters. For all these reasons, it appears very unlikely that this pathway can serve as a basis for industrial exploitation. Other microorganisms have been described as being marginally capable of naturally producing isobutylene from isovalerate; the yields obtained are even lower than those obtained with *Rhodotorula minuta* (Fukuda et al. (Agric. Biol. Chem. 48 (1984), 1679)).
Isoprene is produced at a significant level in higher plants, such as poplars. The production of isoprene in this context remains however low and the pathway which leads to isoprene production, which is based on the mevalonate-isopentenyl-pyrophosphate pathway, poorly complies with the demands for industrial scale production.

Thus, there is still a need for efficient and environmentally friendly methods of producing alkenes.

The present invention meets this demand by providing a method for producing an alkene comprising the step of converting an alcohol into an alkene by an enzymatic dehydration step.
The conversion preferably makes use of a hydratase enzyme, in particular of an oleate hydratase.

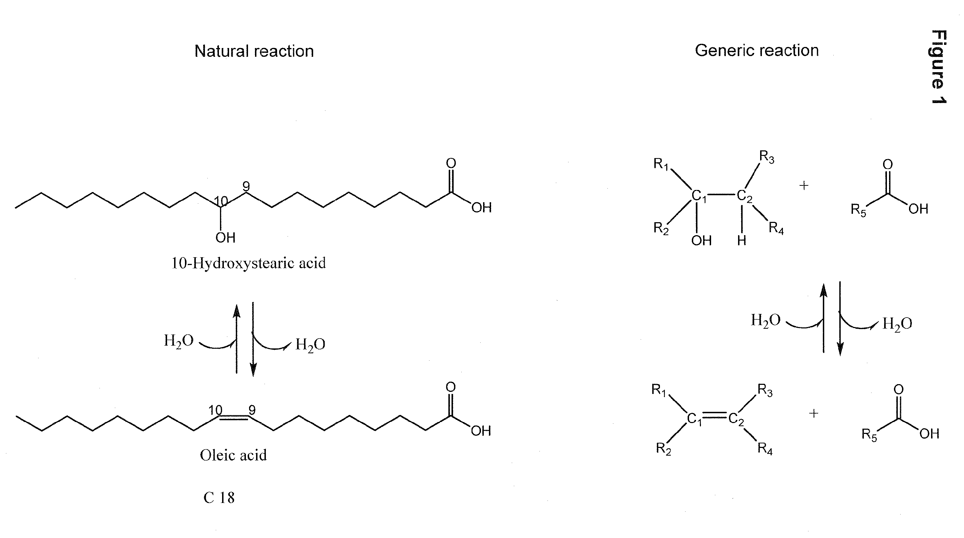

The present invention teaches that it is possible to convert an alcohol enzymatically by the use of a hydratase into an alkene. The enzyme is preferably an oleate hydratase (EC 4.2.1.53).
In particular, it has been found that an enzyme with the activity of an oleate hydratase is not only capable of catalyzing the hydration of oleate but is also capable of catalyzing the dehydration of alcohols and not only of longer alcohols but also of short chain alcohols. Thus, it was found that this enzyme can actually be used to convert alcohols into alkenes which allows the cost efficient and environmentally friendly production of alkenes in microorganism or other organisms. Figure 1 shows a scheme of the reaction catalyzed by oleate hydratase and the generic reaction of converting an alcohol into an alkene.

The alcohol to be used in the method according to the invention is a compound of formula (I): 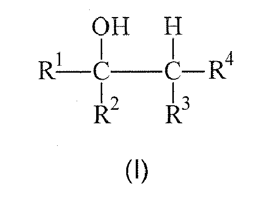 R¹, R², R³ and R⁴ are each independently selected from hydrogen or C₁₋₁₁ hydrocarbyl.

Said hydrocarbyl is preferably selected from alkyl, alkenyl, alkynyl, cycloalkyl, cycloalkenyl, or aryl, more preferably selected from alkyl, alkenyl, alkynyl, or aryl, and even more preferably selected from alkyl, alkenyl, or aryl.

Said alkyl preferably comprises 1 to 11 carbon atoms, more preferably 1 to 8 carbon atoms, more preferably 1 to 6 carbon atoms, and even more preferably 1 to 4 (i.e. 1, 2, 3 or 4) carbon atoms.
Said alkenyl preferably comprises 2 to 11 carbon atoms, more preferably 2 to 8 carbon atoms, more preferably 2 to 6 carbon atoms, and even more preferably 2 to 4 (i.e. 2, 3 or 4) carbon atoms.
Said alkynyl preferably comprises 2 to 11 carbon atoms, more preferably 2 to 8 carbon atoms, more preferably 2 to 6 carbon atoms, and even more preferably 2 to 4 (i.e. 2, 3 or 4) carbon atoms.
Said cycloalkyl preferably comprises 3 to 11 carbon atoms, more preferably 3 to 8 carbon atoms, and even more preferably 3 to 6 (i.e. 3, 4, 5 or 6) carbon atoms.
Said aryl is preferably selected from phenyl or naphthyl, and is more preferably phenyl.

Accordingly, said C₁₋₁₁ hydrocarbyl is preferably selected from C₁-₁₁ alkyl (in particular, C₁₋₆ alkyl or C₁₋₄ alkyl), C₂₋₁₁ alkenyl (in particular, C₂₋₆ alkenyl or C₂₋₄ alkenyl), C₂₋₁₁ alkynyl (in particular, C₂₋₆ alkynyl or C₂₋₄ alkynyl), C₃₋₁₁ cycloalkyl (in particular, C₃₋₆ cycloalkyl), C₃₋₁₁ cycloalkenyl (in particular, C₃₋₆ cycloalkenyl), phenyl or naphthyl. More preferably, said C₁₋₁₁ hydrocarbyl is selected from C₁₋₁₁ alkyl (in particular, C₁₋₆ alkyl or C₁₋₄ alkyl), C₂₋₁₁ alkenyl (in particular, C₂₋₆ alkenyl or C₂₋₄ alkenyl) or phenyl.

In a preferred embodiment, R¹, R², R³, and R⁴ are each independently selected from hydrogen, C₁₋₁₁ alkyl, C₂₋₁₁ alkenyl, C₂₋₁₁ alkynyl, C₃₋₁₁ cycloalkyl, C₃₋₁₁ cycloalkenyl, phenyl or naphthyl, and are more preferably each independently selected from hydrogen, C₁₋₆ alkyl, C₂₋₆ alkenyl or phenyl, and are even more preferably each independently selected from hydrogen or linear C₁₋₄ alkyl.

In general, it is preferred that the compound of formula (I) comprises a total of 2 to 30 carbon atoms, more preferably 2 to 20 carbon atoms, more preferably 2 to 12 carbon atoms, more preferably 2 to 8 carbon atoms, more preferably 2 to 6 carbon atoms, even more preferably 2 to 5 carbon atoms, and most preferably 2, 3 or 4 carbon atoms.

Moreover, while primary, secondary and tertiary alcohols can generally be used in the method of the invention, it is preferred to use a secondary alcohol. Accordingly, it is preferred that one of R¹ and R² comprised in the compound of formula (I) is hydrogen and the other one of R¹ and R² is C₁₋₁₁ hydrocarbyl (or one of the preferred C₁₋₁₁ hydrocarbyl groups described and defined herein above).

It is further preferred that the alcohol to be used in the method according to the invention is a linear (i.e. unbranched) alcohol. Accordingly, it is preferred that one of R¹ and R² in the compound of formula (I) is hydrogen, the other one of R¹ and R² is linear C₁₋₁₁ hydrocarbyl (or one of the preferred C₁₋₁₁ hydrocarbyl groups described and defined herein above which are linear, such as e.g. linear C₁₋₄ alkyl or linear C₂₋₄ alkenyl), one of R³ and R⁴ in the compound of formula (I) is hydrogen, and the other one of R³ and R⁴ is linear C₁₋₁₁ hydrocarbyl (or one of the preferred C₁₋₁₁ hydrocarbyl groups described and defined herein above which are linear, such as e.g. linear C₁₋₄ alkyl or linear C₂₋₄ alkenyl).

It is particularly preferred that the alcohol (or, accordingly, the compound of formula (I)) is selected from: ethanol; propanol, including propan-1-ol (i.e. n-propanol) and propan-2-ol (i.e. isopropanol); butanol, including butan-1-ol (i.e. n-butanol), 2-methylpropan-1-ol (i.e. isobutanol), 2-methylpropan-2-ol (i.e. tert-butanol) and butan-2-ol (i.e. sec-butanol); or pentanol, including pentan-1-ol (i.e. n-pentanol or amyl alcohol), 3-methylbutan-1-ol (i.e. isopentanol or isoamyl alcohol), pentan-2-ol (i.e. sec-pentanol), pentan-3-ol, 2-methylbutan-1-ol, 3-methylbutan-2-ol (i.e. 1,2-dimethylpropanol) and 2-methylbutan-2-ol (i.e. 1,1-dimethylpropanol).

The following Table 1 gives an overview over some examples of alcohols to be employed in the method according to the invention and the resulting alkenes:

**Table 1**

| **Alcohol** | **Alkene** |
|---|---|
| ethanol | ethene (i.e. ethylene) |
| propan-1-ol (i.e. n-propanol) | propene (i.e. propylene) |
| propan-2-ol (i.e. isopropanol) | propene (i.e. propylene) |
| butan-1-ol (i.e. n-butanol) | but-1-ene (i.e. α-butylene) |
| 2-methylpropan-1-ol (i.e. isobutanol) | 2-methylprop-1-ene (i.e. isobutylene) |
| 2-methylpropan-2-ol (i.e. tert-butanol) | 2-methylprop-1-ene (i.e. isobutylene) |
| butan-2-ol (i.e. sec-butanol) | but-1-ene (i.e. α-butylene) |
| butan-2-ol (i.e. sec-butanol) | but-2-ene (i.e. β-butylene) |
| pentan-1-ol (i.e. n-pentanol) | pent-1-ene (i.e. amylene) |
| 3-methylbutan-1-ol (i.e. isopentanol) | 3-methylbut-1-ene |
| pentan-2-ol (i.e. sec-pentanol) | pent-1-ene (i.e. amylene) |
| pentan-2-ol (i.e. sec-pentanol) | pent-2-ene (i.e. β-amylene) |
| pentan-3-ol | pent-2-ene (i.e. β-amylene) |
| 2-methylbutan-1-ol | 2-methylbut-1-ene |
| 3-methylbutan-2-ol (i.e. 1,2-dimethylpropanol) | 2-methylbut-2-ene |
| 3-methylbutan-2-ol (i.e. 1,2-dimethylpropanol) | 3-methylbut-1-ene |
| 2-methylbutan-2-ol (i.e. 1,1-dimethylpropanol) | 2-methylbut-1-ene |
| 2-methylbutan-2-ol (i.e. 1,1-dimethylpropanol) | 2-methylbut-2-ene |
| 3-methylbut-3-en-1-ol | 2-methylbuta-1,3-diene (i.e. isoprene) |
| 2-methylbut-3-en-2-ol | 2-methylbuta-1,3-diene (i.e. isoprene) |
| 2-methylbut-3-en-2-ol | 3-methylbuta-1,2-diene |
| 2-methylbut-3-en-1-ol | 2-methylbuta-1,3-diene (i.e. isoprene) |
| 3-methylbut-3-en-2-ol | 2-methylbuta-1,3-diene (i.e. isoprene) |
| but-3-en-1-ol | buta-1,3-diene |
| but-3-en-2-ol | buta-1,3-diene |
| 1-phenylethanol | styrene (i.e. vinylbenzene) |
| 2-phenylethanol | styrene (i.e. vinylbenzene) |

In one particularly preferred embodiment the alcohol is ethanol and the produced alkene is ethene (i.e. ethylene). In another particularly preferred embodiment the alcohol is propan-2-ol (i.e. isopropanol) and the produced alkene is propene (i.e. propylene). Yet, in a further particularly preferred embodiment the alcohol is butanol and the produced alkene is butene (i.e. butylene). In this case butanol may be any of n-butanol, tert-butanol, sec-butanol or isobutanol. The produced alkene is either but-1-ene (i.e. α-butylene), 2-methylpropene (i.e. isobutylene), or both but-1-ene (i.e. α-butylene) and but-2-ene (i.e. β-butylene). Most preferred in this context are n-butanol and isobutanol.

In a further particularly preferred embodiment the alcohol is any one of 3-methylbut-3-en-1-ol, 2-methylbut-3-en-2-ol, 2-methylbut-3-en-l-ol, 3-methylbut-3-en-2-ol, or mixtures thereof (preferably the alcohol is any one of 3-methylbut-3-en-1-ol, 2-methylbut-3-en-1-ol, 3-methylbut-3-en-2-ol, or mixtures thereof) and the produced alkene is 2-methylbuta-1,3-diene (i.e. isoprene). In another particularly preferred embodiment the alcohol is any one of but-3-en-1-ol, but-3-en-2-ol, or a mixture thereof and the produced alkene is buta-1,3-diene. In another particularly preferred embodiment the alcohol is any one of 1-phenylethanol, 2-phenylethanol, or a mixture thereof and the produced alkene is styrene (i.e. vinylbenzene).

It is to be understood that the alcohol to be used in the method according to the invention may also be a mixture of different compounds of formula (I).

With respect to the product of the method according to the invention, the term "alkene" refers to a compound comprising at least one carbon-to-carbon double bond and, in particular, refers to a compound of formula (II) 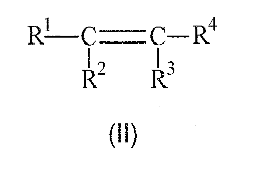 wherein R¹, R², R³, and R⁴ have the same meanings and preferred meanings as described and defined herein above for the compound of formula (I).

It is to be understood that all stereoisomers of the compound of formula (I) can be used in the method according to the invention, either in admixture or in pure or substantially pure form. Furthermore, the compound of formula (II) encompasses both cis-isomers and trans-isomers.

As used herein and unless defined otherwise, the term "hydrocarbyl" refers to a monovalent hydrocarbon group, i.e. a group consisting of carbon atoms and hydrogen atoms, which group is connected to the remainder of the compound of formula (I) via a carbon-to-carbon single bond and may be saturated or unsaturated, linear, branched or cyclic, aliphatic or aromatic. A "C₁₋₁₁ hydrocarbyl" denotes a hydrocarbyl having 1 to 11 carbon atoms.

As used herein and unless defined otherwise, the term "alkyl" refers to a monovalent saturated aliphatic (i.e. non-aromatic) acyclic hydrocarbon group which may be linear or branched and does not comprise any carbon-to-carbon double bond or any carbon-to-carbon triple bond.

As used herein and unless defined otherwise, the term "alkenyl" refers to a monovalent unsaturated aliphatic acyclic hydrocarbon group which may be linear or branched and comprises at least one carbon-to-carbon double bond while it does not comprise any carbon-to-carbon triple bond.

As used herein and unless defined otherwise, the term "alkynyl" refers to a monovalent unsaturated aliphatic acyclic hydrocarbon group which may be linear or branched and comprises at least one carbon-to-carbon triple bond and optionally one or more carbon-to-carbon double bonds.

As used herein and unless defined otherwise, the term "cycloalkyl" refers to a monovalent cyclic saturated aliphatic hydrocarbon group which does not comprise any carbon-to-carbon double bond or any carbon-to-carbon triple bond. Non-limiting examples of cycloalkyl groups are cyclopropyl, cyclobutyl, cyclopentyl or cyclohexyl.

As used herein and unless defined otherwise, the term "cycloalkenyl" refers to a monovalent cyclic unsaturated aliphatic hydrocarbon group which comprises at least one carbon-to-carbon double bond and does not comprise any carbon-to-carbon triple bond. Non-limiting examples of cycloalkyl groups are cyclopropenyl, cyclobutenyl, cyclopentenyl, cyclohexenyl or cyclohexadienyl.

As used herein and unless defined otherwise, the term "aryl" refers to a monovalent aromatic hydrocarbon group, including bridged ring and/or fused ring systems, containing at least one aromatic ring. "Aryl" may, for example, refer to phenyl, naphthyl or anthracenyl.

An enzyme with the activity of a hydratase is an enzyme which is capable of catalyzing the hydration-dehydration of C-O linkages. In general, such an enzyme can also catalyze the reverse reaction, i.e. the dehydration. In the scope of dehydration such an enzyme catalyzes the elimination of oxygen and hydrogen from organic compounds in the form of water.
In general, in the context of the present invention any enzyme could be used which has hydratase activity. In a preferred embodiment the hydratase enzyme is a fatty acid hydratase. A fatty acid hydratase is understood to be an enzyme which has the capacity to catalyse the addition of a water molecule to an unsaturated fatty acid. The term "unsaturated fatty acid" refers to a fatty acid which contains at least one carbon-to-carbon double bond. In principle the unsaturated fatty acid can be any unsaturated fatty acid. Preferably the unsaturated fatty acid is a fatty acid which has more than 4, more than 6, more than 8, more than 10, more than 12 or more than 14 carbon atoms. In another preferred embodiment the unsaturated fatty acid has not more than 28, not more than 26, not more than 24, not more than 22, not more than 20 or not more than 18 carbon atoms. In a preferred embodiment the unsaturated fatty acid has a length between 10 and 22 carbon atoms. Examples for unsaturated fatty acids include obtusilic acid (C10:1), caproleic acid (C10:1), lauroleic acid (C12:1), linderic acid (C12:1), myristoleic acid (C14:1), physeteric acid (C14:1), tsuzuic acid (C14:1), palmitoleic acid (C 16:1), sapienic acid (C16:1), oleic acid (C18:1), linoleic acid (C18:2), alpha-linolenic acid (C18:3), petroselinic acid (C18:1), elaidic acid (C18:1), vaccenic acid (C18:1), arachidonic acid (C20:4), eicosapentanoic acid (C20:5), gadoleic acid (C20:1), gondoic acid (C20:1), erucic acid (C22:1), docosahexaenoic acid (C22:6), cetoleic acid (C22:1), erucic acid (C22:1) and nervonic acid (C24:1), and in particular lauroleic acid (C12:1), myristoleic acid (C14:1), palmitoleic acid (C 16:1), oleic acid (C18:1), linoleic acid (C18:2), alpha-linolenic acid (C18:3), arachidonic acid (C20:4), eicosapentanoic acid (C20:5), erucic acid (C22:1) and docosahexaenoic acid (C22:6). In a preferred embodiment the fatty acid is a fatty acid with a length of between 10 and 22 carbon atoms and which has a double bond between carbon atoms 9 and 10. It is to be understood that the fatty acid may also be present in a deprotonated form; for example, oleic acid may be present in its deprotonated form as the anion oleate.
Thus, in a particularly preferred embodiment the fatty acid hydratase is an enzyme which is capable of catalysing the addition of a water molecule to an unsaturated fatty acid which has a double bond between carbon atoms 9 and 10 and which leads to the formation of a 10-hydroxy fatty acid. In an even more preferred embodiment the fatty acid is oleate. For numbering the C-atoms within the fatty acid molecules the usual numbering is used starting from the carbon of the carboxy group of the fatty acid.
The hydratase and in particular the fatty acid hydratase activity can be measured by incubating the corresponding enzyme with the corresponding substrate, in particular an unsaturated fatty acid substrate, under appropriate conditions and analysing the reaction products, e.g. by GC-MS analysis.
In a particularly preferred embodiment the fatty acid hydratase is an oleate hydratase. In the context of the present invention the term "oleate hydratase" or "an enzyme with the activity of an oleate hydratase" is understood to be an enzyme which is capable of catalyzing the hydration of a non-activated carbon-carbon bond. In particular, such an enzyme is capable of converting oleic acid (OA) into 10-hydroxystearic acid (10-HAS) via hydration. This enzymatic activity can be measured by incubating the enzyme with oleic acid and analysing the reaction products, e.g. via gas chromatography. It can in particular be assayed, e.g., by an assay as described in Bevers et al. (J. Bacteriol. 191 (2009), 5010-5012). In brief, the enzyme is incubated for at least 1 h in 20 mM Tris (pH 8)-50 mM NaCl with OA at an appropriate temperature at which the enzyme shows activity (e.g., 22°C, 30°C or 37°C). The reaction is stopped by the addition of 3M HCl and the products are analysed by gas chromatography (GC). The occurrence of 10-HAS is indicative of oleate hydratase activity. Alternatively, the activity can also be tested as described in WO 2008/119735. In this assay 20 µg oleic acid is mixed with the enzyme to be tested in 1 ml 0.1 M sodium phosphate pH 7.1, 0.1 M NaCl and incubated for at least 1 h at an appropriate temperature (e.g., 22°C, 30°C or 37°C). Subsequently the products are characterized by GC (see Example 6 of WO 2008/119735).
Preferably, in the context of the present application the term "oleate hydratase" or "a protein/enzyme having the activity of an oleate hydratase" refers to any enzyme which is classified in the EC number EC 4.2.1.53.
In principle any hydratase enzyme and in particular any oleate hydratase enzyme can be used in the context of the present invention, in particular any hydratase enzyme or oleate hydratase enzyme which is derived from a prokaryotic or a eukaryotic organism. Oleate hydratase has been described from different organisms, e.g. from Streptococcus pyogenes (WO 2008/119735; SEQ ID NO:1) and from Elizabethkingia meningoseptica (Bevers et al., loc. cit.; GenBank Accessionnumber GQ144652; SEQ ID NO:2). It has been found that about 165 homologues to the Elizabethkingia meningoseptica sequence can be found in other organisms (see Bevers et al., loc. cit.). Originally most of these proteins were annotated as cross-myosin-reactive antigen due to their homology to the Streptococcus pyogenes 67.5 kDa protein which had later been found to have oleate hydratase activity (WO 2008/119735). Examples for such sequences are shown in SEQ ID NOs: 3 to 11.
Moreover, the following Table 2 lists some of the proteins found to be homologous to the above mentioned protein from Elizabethkingia meningoseptica. These proteins constitute further preferred embodiments of enzymes employed in the method according to the present invention.

**Table 2**

| Uniprot accession number | Organism |
|---|---|
| C7DLJ6 | Flavobacterium meningosepticum (Elizabethkingia menigoseptica) |
| Q1VYG7 | Psychroflexus torquis ATCC 700755. |
| Q6N2EO | Rhodopseudomonas palustris |
| A9W1Y8 | Methylobacterium extorquens (strain PA1). |
| B2TM67 | Clostridium botulinum (strain Eklund 17B / Type B). |
| A316F1 | Bacillus sp. B14905. |
| B9YB92 | Holdemania filiformis DSM 12042. |
| Q5FL96 | Lactobacillus acidophilus. |
| A6QD90 | Staphylococcus aureus (strain Newman) |
| Q5XDG8 | Streptococcus pyogenes serotype M6. |
| C7ZMN5 | Nectria haematococca (strain 77-13-4 / FGSC 9596 / MPVI) |
| Q8XNF5 | Clostridium perfringens. |
| C7WF65 | Enterococcus faecalis DS5. |
| Q8G7V6 | Bifidobacterium longum. |
| C7LRDO | Desulfomicrobium baculatum (strain DSM 4028 / VKM B-1378) |
| B7AW97 | Bacteroides pectinophilus ATCC 43243. |
| C4C3A3 | Sebaldella termitidis ATCC 33386 |
| Q5FNW1 | Gluconobacter oxydans |
| C6PA43 | Thermoanaerobacterium thermosaccharolyticum DSM 571. |
| C7L9A6 | Acetobacter pasteurianus IFO 3283-12. |
| Q5V130 | Haloarcula marismortui |
| A6TJY6 | Alkaliphilus metalliredigens (strain QYMF). |
| B2UE67 | Ralstonia pickettii (strain 12J). |
| C3RJT3 | Mollicutes bacterium D7. |

In a preferred embodiment of the present invention the oleate hydratase is an enzyme comprising an amino acid sequence selected from the group consisting of SEQ ID NOs: 1 to 11 or a sequence which is at least n % identical to any of SEQ ID NOs: 1 to 11 and having the activity of an oleate hydratase with n being an integer between 10 and 100, preferably 10, 15, 20, 25, 30, 35, 40, 45, 50, 55, 60, 65, 70, 75, 80, 85, 90, 91, 92, 93, 94, 95, 96, 97, 98 or 99. The term "sequence identity" preferably means the same amino acid residues in the same N- to C-terminal direction.
Preferably, the degree of identity is determined by comparing the respective sequence with the amino acid sequence of any one of the above-mentioned SEQ ID NOs. When the sequences which are compared do not have the same length, the degree of identity preferably either refers to the percentage of amino acid residues in the shorter sequence which are identical to amino acid residues in the longer sequence or to the percentage of amino acid residues in the longer sequence which are identical to amino acid residues in the shorter sequence. The degree of sequence identity can be determined according to methods well known in the art using preferably suitable computer algorithms such as CLUSTAL.
When using the Clustal analysis method to determine whether a particular sequence is, for instance, 80% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.
Other algorithms which can be used for calculating sequence identity are those of Needleman and Wunsch or of Smith and Watermann. For sequence comparisons the program PileUp (Feng and Doolittle, J. Mol. Evolution 25 (1987), 351-360; Higgins et al., CABIOS 5 (1989), 151-153) or the programs Gap and Best Fit (Needleman and Wunsch, J. Mol. Biol. 48 (1970), 443-453; Smith and Waterman, Adv. Appl. Math. 2 (1981), 482-489) can be used, which are contained in the GCG software package (Genetics Computer Group, 575 Science Drive, Madison, Wisconsin, USA). Preferably, the settings which are used are the standard settings for sequence comparisons.
Preferably, the degree of identity is calculated over the complete length of the sequence.

The hydratase, preferably the oleate hydratase, employed in the process according to the invention can be a naturally occurring enzyme or it can be an enzyme which is derived from a naturally occurring hydratase, preferably oleate hydratase, e.g. by the introduction of mutations or other alterations which, e.g., alter or improve the enzymatic activity, the stability, etc.
The term "hydratase" or "a protein/enzyme having the activity of a hydratase" in the context of the present application also covers enzymes which are derived from a hydratase, which are capable of dehydrating an alcohol as defined above into an alkene but which only have a low affinity to their natural substrate or do no longer accept their natural substrate.
Accordingly, the term "oleate hydratase" or "a protein/enzyme having the activity of an oleate hydratase" in the context of the present application also covers enzymes which are derived from an oleate hydratase, which are capable of dehydrating an alcohol as defined above into an alkene but which only have a low affinity to oleate as a substrate or do no longer accept oleate as a substrate. Such a modification of the preferred substrate of an oleate hydratase allows to improve the conversion of the alcohol into the alkene and to reduce the production of unwanted by-product due to the action of the enzyme on oleate or other unsaturated fatty acids. Methods for modifying and/or improving the desired enzymatic activities of proteins are well-known to the person skilled in the art and include, e.g., random mutagenesis or site-directed mutagenesis and subsequent selection of enzymes having the desired properties or approaches of the so-called "directed evolution".
For example, for genetic engineering in prokaryotic cells, a nucleic acid molecule encoding a hydratase, preferably an oleate hydratase, can be introduced into plasmids which permit mutagenesis or sequence modification by recombination of DNA sequences. Standard methods (see Sambrook and Russell (2001), Molecular Cloning: A Laboratory Manual, CSH Press, Cold Spring Harbor, NY, USA) allow base exchanges to be performed or natural or synthetic sequences to be added. DNA fragments can be connected to each other by applying adapters and linkers to the fragments. Moreover, engineering measures which provide suitable restriction sites or remove surplus DNA or restriction sites can be used. In those cases, in which insertions, deletions or substitutions are possible, *in vitro* mutagenesis, "primer repair", restriction or ligation can be used. In general, a sequence analysis, restriction analysis and other methods of biochemistry and molecular biology are carried out as analysis methods. The resulting hydratase, preferably oleate hydratase variants are then tested for their enzymatic activity and in particular for their capacity to dehydrate alcohols and prefer them as a substrate rather than their natural substrate, e.g. fatty acids and in particular oleate.
An assay for measuring the capacity of an oleate hydratase to dehydrate an alcohol and to convert it into an alkene is describe in Examples, in particular Examples 2 to 8.

Methods for identifying variants with improved enzymatic properties as regards the production of alkenes may also be carried out in the presence of a "cofactor" which allows for a steric and/or electronic complementation in the catalytic site of the enzyme due to the fact that the alcohol used as a substrate may be shorter than the fatty acid which functions as the natural substrate, e.g. oleate. The cofactor may depend on the natural substrate of the enzyme to be employed in the method according to the invention. The following Table 3 summarizes the substrates, products and possible cofactors.

**Table 3**

| **Alcohol** | **Alkene** | **Possible cofactor** |
|---|---|---|
| ethanol | ethene (i.e. ethylene) | octanoic acid |
| propan-1-ol (i.e. n-propanol) | propene (i.e. propylene) | heptanoic acid |
| propan-2-ol (i.e. isopropanol) | propene (i.e. propylene) | octanoic acid |
| butan-1-ol (i.e. n-butanol) | but-1-ene (i.e. α-butylene) | hexanoic acid |
| 2-methylpropan-1-ol (i.e. isobutanol) | 2-methylprop-1-ene (i.e. isobutylene) | heptanoic acid |
| 2-methylpropan-2-ol (i.e. tert-butanol) | 2-methylprop-1-ene (i.e. isobutylene) | octanoic acid |
| butan-2-ol (i.e. sec-butanol) | but-1-ene (i.e. α-butylene) | octanoic acid |
| butan-2-ol (i.e. sec-butanol) | but-2-ene (i.e. β-butylene) | heptanoic acid |
| pentan-1-ol (i.e. n-pentanol) | pent-1-ene (i.e. amylene) | pentanoic acid |
| 3-methylbutan-1-ol (i.e. isopentanol) | 3-methylbut-1-ene | hexanoic acid |
| pentan-2-ol (i.e. sec-pentanol) | pent-1-ene (i.e. amylene) | octanoic acid |
| pentan-2-ol (i.e. sec-pentanol) | pent-2-ene (i.e. β-amylene) | hexanoic acid |
| pentan-3-ol | pent-2-ene (i.e. β-amylene) | heptanoic acid |
| 2-methylbutan-1-ol | 2-methylbut-1-ene | hexanoic acid |
| 3-methylbutan-2-ol (i.e. 1,2-dimethylpropanol) | 2-methylbut-2-ene | heptanoic acid |
| 3-methylbutan-2-ol (i.e. 1,2-dimethylpropanol) | 3-methylbut-1-ene | octanoic acid |
| 2-methylbutan-2-ol (i.e. 1,1-dimethylpropanol) | 2-methylbut-1-ene | octanoic acid |
| 2-methylbutan-2-ol (i.e. 1,1dimethylpropanol) | 2-methylbut-2-ene | heptanoic acid |
| 3-methylbut-3-en-1-ol | 2-methylbuta-1,3-diene (i.e. isoprene) | hexanoic acid |
| 2-methylbut-3-en-2-ol | 2-methylbuta-1,3-diene (i.e. isoprene) | octanoic acid |
| 2-methylbut-3-en-2-ol | 3-methylbuta-1,2-diene | heptanoic acid |
| 2-methylbut-3-en-1-ol | 2-methylbota-1,3-diene (i.e. isoprene) | hexanoic acid |
| 3-methylbut-3-en-2-ol | 2-methylbuta-1,3-diene (i.e. isoprene) | octanoic acid |
| but-3-en-1-ol | buta-1,3-diene | hexanoic acid |
| but-3-en-2-ol | buta-1,3-diene | heptanoic acid |
| 1-phenylethanol | styrene (i.e. vinylbenzene) | octanoic acid |
| 2-phenylethanol | styrene (i.e. vinylbenzene) | hexanoic acid or heptanoic acid |

Preferred cofactors are linear alkanoic acids of the general formula H-(CH2)n-COOH having 2 to 8 carbon atoms, preferably 3 to 8, even more preferably 3 to 6 carbon atoms. Also conceivable as cofactors are analogs and specifically alkane sulfonic acids of the general formula H-(CH2)n-SO3H.

The modified version of the hydratase, preferably oleate hydratase, accepting an alcohol as a substrate but having a low affinity to its natural substrate, e.g.a fatty acid, in particular oleate, as a substrate or no longer accepting its natural substrate, preferably a fatty acid, in particular oleate, as a substrate may be derived from a naturally occurring hydratase, preferably oleate hydratase, or from an already modified, optimized or synthetically produced hydratase, preferably oleate hydratase.

The enzyme employed in the process according to the present invention can be a natural version of the protein or a synthetic protein as well as a protein which has been chemically synthesized or produced in a biological system or by recombinant processes. The enzyme may also be chemically modified, for example in order to improve its/their stability, resistance, e.g. to temperature, for facilitating its purification or its immobilization on a support. The enzyme may be used in isolated form, purified form, in immobilized form, as a crude or partially purified extract obtained from cells synthesizing the enzyme, as chemically synthesized enzyme, as recombinantly produced enzyme, in the form of microorganisms producing them etc.

The process according to the present invention may be carried out in vitro or in vivo. An in vitro reaction is understood to be a reaction in which no cells are employed, i.e. an acellular reaction.
For carrying out the process in vitro the substrates for the reaction and the enzyme are incubated under conditions (buffer, temperature, cofactors etc.) allowing the enzyme to be active and the enzymatic conversion to occur. The reaction is allowed to proceed for a time sufficient to produce alkene. The production of alkene can be detected by gas chromatography (GC) or GC/MS analysis.
The enzyme may be in any suitable form allowing the enzymatic reaction to take place. It may be purified or partially purified or in the form of crude cellular extracts or partially purified extracts. It is also possible that the enzyme is immobilized on a suitable carrier.
Since the alcohol used as a substrate may be shorter than the natural substrate used by the enzyme, e.g. oleate, it may be advantageous to add to the reaction mixture a "cofactor" which allows for a steric and/or electronic complementation in the catalytic site of the enzyme as mentioned above.

In general, if the alkene product is a gaseous and scarcely soluble in water under the conditions of temperature at which the process is conducted, the equilibrium of the reaction catalyzed by the enzyme employed is shifted and the reaction goes to completion in the direction of the formation of the gasous alkene , in particular if that gas is permanently removed from the reaction vessel.

In one particularly preferred embodiment, the hydratase enzyme used in the process according to the invention is a thermophilic hydratase, i.e. a hydratase which is capable of catalyzing the reaction at elevated temperatures. The term "elevated temperatures" means temperatures above 37°C. Such enzymes can either be isolated from corresponding thermophilic organisms, in particular bacteria or archaebacteria, or they can be obtained by mutagenizing available hydratase sequences and testing them for an increased enzymatic activity under increased temperature conditions. The advantage of using a hydratase which is functional at elevated temperatures is that the produced alkene can immediately go into the gaseous phase and can be constantly removed from the reaction thereby driving the reaction into the direction of dehydration. This advantage exists for all the produced alkenes which are in gaseous form at or below the temperature at which the reaction is carried out. Accordingly, in the method of the present invention the step of converting an alcohol by an enzymatic dehydration step into an alkene is preferably carried out at an elevated temperature (i.e. at a temperature above 37°C, including a temperature above 37°C and below 100°C, such as, e.g., at a temperature of 38°C, 40°C, 50°C, 70°C or 90°C) and the enzymatic dehydration step is catalyzed by a thermophilic hydratase as described herein above. The use of elevated temperatures also allows to produce longer alkenes in a manner that they directly degas from the reaction mixture, e.g. alkenes having 6 or 7 carbon atoms. Thus, in this preferred embodiment the alkene to be produced has preferably at least 4, even more preferably at least 5 even more preferably at least 6 and most preferably at least 7 carbon atoms. Moreover, it is preferred that the alkene does not have more than 12, 10 or 8 carbon atoms.

For carrying out the process in vivo use is made of a suitable organism/microorganism which is capable of expressing a hydratase enzyme as defined above. In principle, the substrate, i.e. the alcohol to be converted into the alkene, can be provided externally to the microorganism. However, in a preferred embodiment the organism/microorganism is also capable of producing the substrate, i.e. the alcohol to be converted.
Thus, in the case of this embodiment the method according to the invention is characterised in that the conversion of the alcohol into the alkene is realized in the presence of an organism/ microorganism capable of expressing a hydratase enzyme as defined above and preferably also capable of producing an alcohol which should be converted.

The term "which is capable of producing an alcohol" in the context of the present invention means that the organism/microorganism has the capacity to produce such an alcohol within the cell due to the presence of enzymes providing enzymatic activities allowing the production of such an alcohol from metabolic precursors. The organism/microorganism can be an organism/microorganism which naturally has the capacity to produce the corresponding alcohol or it can be an organism/microorganism which has been genetically modified so as to be capable of producing the corresponding alcohol.

For example, in one preferred embodiment of the present invention the alcohol to be converted into an alkene (in particular ethylene) is ethanol and the organism/microorganism employed in the method according to the invention is an organism/microorganism which is capable of producing ethanol. Examples for microorganism which naturally produce ethanol are yeast, in particular Saccharomyces species, Klyveromyces lactis and bacteria of the genus Zymomonas, most preferably Zymomonas mobilis. However, according to this embodiment any organism can be used which has the capacity to produce ethanol. In particular, it is also possible to use an organism/microorganism which has been genetically modified so as to confer to it the capacity to produce ethanol.

In another preferred embodiment of the present invention the alcohol to be converted into an alkene (in particular α-butylene) is n-butanol and the organism/microorganism employed in the method according to the invention is an organism/microorganism which is capable of producing n-butanol. Examples for microorganism which naturally produce n-butanol are bacteria of the genus Clostridium, preferably Clostridium acetobutylicum. However, it is also possible to use an organism/microorganism which has been genetically modified so as to confer to it the capacity to produce n-butanol.

In another preferred embodiment of the present invention the alcohol to be converted into an alkene (in particular isobutylene) is isobutanol and the organism/microorganism employed in the method according to the invention is an organism/microorganism which is capable of producing isobutanol. Examples for microorganism which produce isobutanol are bacteria of the genus E. coli which have been genetically modified so as to produce isobutanol (Connor and Liao (Curr. Opin. Biotechnol. 20 (2009), 307). Another example of a microorganism which can produce isobutanol is S. cerevisiae. In particular, S. cerevisiae is capable of producing isobutanol when fed with valine as a carbon source (Dickinson et al. (J. Biol. Chem. 273 (1998), 25751)). Another organism which is capable of producing isobutanol is Lactococcus lactis.

In yet a further preferred embodiment according to the present invention the alcohol to be converted into an alkene (in particular propylene) is isopropanol and the organism/microorganism employed in the method according to the invention is an organism/microorganism which is capable of producing isopropanol. Examples for microorganisms which are capable of producing isopropanol are acetone-producing bacteria which have been equipped with a secondary alcohol dehydrogenase which converts acetone into isopropanol. In particular, when a secondary alcohol dehydrogenase is produced in these bacteria the reaction (in the reverse orientation converts acetone into isopropanol that can then be further converted into isopropanol according to the method of the present invention. Corresponding E. coli cells are described in Jojima et al. (Appl. Microbiol. Biotechnol. 77 (2008), 1219-1224) and in Hanai et al. (Appl. Environ. Microbiol. 73 (2007), 7814-7818). A source for the secondary alcohol dehydrogenase is, e.g. Clostridium beijerinckii.

Acetone is produced by certain microorganisms, such as Clostridium acetobutylicum, Clostridium beijerinckii, Clostridium cellulolyticum, Bacillus polymyxa and Pseudomonas putida. The synthesis of acetone is best characterized in Clostridium acetobutylicum. It starts out with a reaction (reaction step 1) in which two molecules of acetyl-CoA are condensed into acetoacetyl-CoA. This reaction is catalyzed by acetyl-CoA acetyltransferase (EC 2.3.1.9). Acetoacetyl-CoA is then converted into acetoacetate by a reaction with acetic acid or butyric acid resulting also in the production of acetyl-CoA or butyryl-CoA (reaction step 2). This reaction is catalyzed e.g. by acetoacetylCoA transferase (EC 2.8.3.8). AcetoacetylCoA transferase is known from various organisms, e.g. from E. coli in which it is encoded by the atoAD gene or from Clostridium acetobutylicum in which it is encoded by the ctfAB gene. However, also other enzymes can catalyze this reaction, e.g. 3-oxoacid CoA transferase (EC 2.8.3.5) or succinate CoA ligase (EC 6.2.1.5).
Finally, acetoacetate is converted into acetone by a decarboxylation step (reaction step 3) catalyzed by acetoacetate decarboxylase (EC 4.1.1.4).

The above described reaction steps 1 and 2 and the enzymes catalyzing them are not characteristic for the acetone synthesis and can be found in various organism. In contrast, reaction step 3 which is catalyzed by acetoacetate decarboxylase (EC 4.1.1.4) is only found in those organisms which are capable of producing acetone. The prior art already describes microorganisms which have been genetically modified so as to be able to produce acetone. In particular genes from, e.g., Clostridium acetobutylicum have been introduced into E. coli thereby allowing the synthesis of acetone in E. coli, a bacterium which naturally does not produce acetone (Bermejo et al., Appl. Environ. Microbiol. 64 (1998); 1079-1085; Hanai et al., Appl. Environ. Microbiol. 73 (2007), 7814-7818). In particular Hanai et al. (loc. cit.) shows that it is sufficient to introduce a nucleic acid sequence encoding an acetoacetate decarboxylase (such as that from Clostridium acetobutylicum) in order to achieve acetone production in E. coli indicating that the endogenous enzymes in E. coli catalyzing the above-mentioned reaction steps 1 and 2 (i.e. the expression products of the E. coli atoB and atoAD genes) are sufficient to provide substrate for the acetone production.

In one preferred embodiment the organism employed in the method according to the invention is an organism, preferably a microorganism, which naturally has the capacity to produce acetone and furthermore expresses a secondary alcohol dehydrogenase so as to convert acetone into isopropanol which can then be further converted into propylene. Thus, preferably the microorganism belongs to the genus Clostridium, Bacillus or Pseudomonas, more preferably to the species Clostridium acetobutylicum, Clostridium beijerinckii, Clostridium cellulolyticum, Bacillus polymyxa or Pseudomonas putida. However, it is, of course, also possible to use an organism/microorganism which has been genetically modified so as to confer to it the capacity to produce acetone. The genes responsible for acetone production have been cloned and also methods for transferring these genes into other organisms, such as E. coli, to confer to them the capacity to produce acetone have already been described.

In a preferred embodiment, the organism employed in the method according to the invention is an organism, preferably a microorganism, which has the capacity to produce the respective alcohol to be converted into the corresponding alkene and which is recombinant in the sense that it has further been genetically modified so as to express a hydratase enzyme as defined above. The term "recombinant" in one embodiment means that the organism is genetically modified so as to contain a foreign nucleic acid molecule encoding a hydratase enzyme as defined above. In a preferred embodiment the organism has been genetically modified so as to contain a foreign nucleic acid molecule encoding a hydratase enzyme as defined above. The term "foreign" in this context means that the nucleic acid molecule does not naturally occur in said organism/microorganism. This means that it does not occur in the same structure or at the same location in the organism/microorganism. In one preferred embodiment, the foreign nucleic acid molecule is a recombinant molecule comprising a promoter and a coding sequence encoding the hydratase enzyme in which the promoter driving expression of the coding sequence is heterologous with respect to the coding sequence. Heterologous in this context means that the promoter is not the promoter naturally driving the expression of said coding sequence but is a promoter naturally driving expression of a different coding sequence, i.e., it is derived from another gene, or is a synthetic promoter or a chimeric promoter. Preferably, the promoter is a promoter heterologous to the organism/microorganism, i.e. a promoter which does not naturally occur in the respective organism/microorganism. Even more preferably, the promoter is an inducible promoter. Promoters for driving expression in different types of organisms, in particular in microorganisms, are well known to the person skilled in the art.
In another preferred embodiment the nucleic acid molecule is foreign to the organism/microorganism in that the encoded hydratase enzyme is not endogenous to the organism/microorganism, i.e. is naturally not expressed by the organism/microorganism when it is not genetically modified. In other words, the encoded hydratase enzyme is heterologous with respect to the organism/microorganism.
The term "recombinant" in another embodiment means that the organism is genetically modified in the regulatory region controlling the expression of a hydratase enzyme as defined above which naturally occurs in the organism so as to lead to an increase in expression of the respective enzyme in comparison to a corresponding non-genetically modified organism. The meaning of the term high "higher expression" is described further below.

Such a modification of a regulatory region can be achieved by methods known to the person skilled in the art. One example is to exchange the naturally occurring promoter by a promoter which allows for a higher expression or to modify the naturally occurring promoter so as to show a higher expression. Thus, in this embodiment the organism contains in the regulatory region of the gene encoding an enzyme as defined above a foreign nucleic acid molecule which naturally does not occur in the organism and which leads to a higher expression of the enzyme in comparison to a corresponding non-genetically modified organism.
The foreign nucleic acid molecule may be present in the organism/microorganism in extrachromosomal form, e.g. as plasmid, or stably integrated in the chromosome. A stable integration is preferred.

In another preferred embodiment the organism/microorganism is characterized in that the expression/activity of a hydratase enzyme as defined above is higher in the organism/microorganism genetically modified with the foreign nucleic acid molecule in comparison to the corresponding non-genetically modified organism/microorganism. A "higher" expression/activity means that the expression/activity of the hydratase enzyme in the genetically modified microorganism is at least 10%, preferably at least 20%, more preferably at least 30% or 50%, even more preferably at least 70% or 80% and particularly preferred at least 90% or 100% higher than in the corresponding non-genetically modified organism/microorganism. In even more preferred embodiments the increase in expression/activity may be at least 150%, at least 200% or at least 500%. In particularly preferred embodiments the expression is at least 10-fold, more preferably at least 100-fold and even more preferred at least 1000-fold higher than in the corresponding non-genetically modified organism/microorganism.
The term "higher" expression/activity also covers the situation in which the corresponding non-genetically modified organism/microorganism does not express a corresponding hydratase enzyme so that the corresponding expression/activity in the non-genetically modified organism/microorganism is zero.
Methods for measuring the level of expression of a given protein in a cell are well known to the person skilled in the art. In one embodiment, the measurement of the level of expression is done by measuring the amount of the corresponding protein. Corresponding methods are well known to the person skilled in the art and include Western Blot, ELISA etc. In another embodiment the measurement of the level of expression is done by measuring the amount of the corresponding RNA. Corresponding methods are well known to the person skilled in the art and include, e.g., Northern Blot.
Methods for measuring the enzymatic activity of the hydratase enzyme are known in the art and have already been described above.

Methods for preparing an organism which is genetically modified so as to produce a hydratase as described above, preferably a microorganism, are well known in the art. Thus, generally, the organism/microorganism is transformed with a DNA construct allowing expression of the respective enzyme in the microorganism. Such a construct normally comprises the coding sequence in question linked to regulatory sequences allowing transcription and translation in the respective host cell, e.g. a promoter and/or enhancer and/or transcription terminator and/or ribosome binding sites etc.

The term "organism" as used in the context of the present invention refers in general to any possible type of organism, in particular eukaryotic organisms, prokaryotic organisms and archaebacteria. The term includes animal, plants, fungi, bacteria and archaebacteria. The term also includes isolated cells or cell aggregates of such organisms, like tissue or calli.
In one preferred embodiment, the organism is a microorganism. The term "microorganism" in the context of the present invention refers to prokaryotic cells, in particular bacteria, as well as to fungi, such as yeasts, and also to algae and archaebacteria. In one preferred embodiment, the microorganism is a bacterium. In principle any bacterium can be used. Preferred bacteria to be employed in the process according to the invention are bacteria of the genus Bacillus, Clostridium, Pseudomonas, Zymomonas or Escherichia. In a particularly preferred embodiment the bacterium belongs to the genus Escherichia and even more preferred to the species Escherichia coli.
In another preferred embodiment the microorganism is a fungus, more preferably a fungus of the genus Saccharomyces, Schizosaccharomyces, Aspergillus or Trichoderma and even more preferably of the species Saccharomyces cerevisiae, Schizosaccharomyces pombe, Aspergillus niger or of the species Trichoderma reesei.

In still another preferred embodiment the microorganism is a photosynthetically active microorganism such as bacteria which are capable of carrying out photosynthesis or micro-algae.
In a particularly preferred embodiment the microorganism is an algae, more preferably an algae belonging to the diatomeae.

If microorganisms are used in the context of the method of the present invention, it is also conceivable to carry out the method according to the invention in a manner in which two types of microorganisms are employed, i.e. one type which produces the alcohol which should be converted into an alkene and one type which uses the alcohol produced by the first type of microorganisms to convert it with the help of a hydratase enzyme as defined herein above into the respective alkene.

When the process according to the invention is carried out in vivo by using microorganisms providing the respective enzyme activity, the microorganisms are cultivated under suitable culture conditions allowing the occurrence of the enzymatic reaction. The specific culture conditions depend on the specific microorganism employed but are well known to the person skilled in the art. The culture conditions are generally chosen in such a manner that they allow the expression of the genes encoding the enzymes for the respective reactions. Various methods are known to the person skilled in the art in order to improve and fine-tune the expression of certain genes at certain stages of the culture such as induction of gene expression by chemical inducers or by a temperature shift.

In another preferred embodiment the organism employed in the method according to the invention is an organism which is capable of photosynthesis, such as a plant or microalgae. In principle any possible plant can be used, i.e. a monocotyledonous plant or a dicotyledonous plant. It is preferable to use a plant which can be cultivated on an agriculturally meaningful scale and which allows to produce large amounts of biomass. Examples are grasses like Lolium, cereals like rye, barley, oat, millet, maize, other starch storing plants like potato or sugar storing plants like sugar cane or sugar beet. Conceivable is also the use of tobacco or of vegetable plants such as tomato, pepper, cucumber, egg plant etc. Another possibility is the use of oil storing plants such as rape seed, olives etc. Also conceivable is the use of trees, in particular fast growing trees such as eucalyptus, poplar or rubber tree (Hevea brasiliensis).

In a particularly preferred embodiment the organism/microorganism employed in the method according to the invention is an organism/microorganism which is thermophilic in the sense that it can survive and catalyze the dehydration of the alcohol in question at elevated temperatures. The term "elevated" temperature means a temperature over 37°C. Examples for such organism/microorganism are bacteria of the genus Thermus, e.g. Thermus thermophilus or Thermus aquaticus, or bacteria of the genus Clostridium, such as Clostridium thermocellum. Other examples are microorganisms which are extremely heat-tolerant, e.g. microorganisms of the genus Thermotoga, such as Thermotoga maritime, or microorganisms of the genus Aquifex, such as Aquifex aeolicus.

The present invention also relates to an organism, preferably a microorganism, which is characterized by the following features:
(a) it is capable of producing an alcohol; and
(b) it expresses a hydratase enzyme which is capable of catalyzing the conversion of the alcohol into an alkene.

As regards the source, nature, properties, sequence etc. of the hydratase enzyme expressed in the organism according to the invention, the same applies as has been set forth above in connection with the method according to the invention.

In one preferred embodiment, the organism according to the invention is an organism, preferably a microorganism, which naturally has the capacity to produce the alcohol, i.e., feature (a) mentioned above is a feature which the organism, preferably microorganism, shows naturally. In a particularly preferred embodiment the alcohol is ethanol, isopropanol, n-butanol or isobutanol.
As regards organisms/microorganisms producing such alcohols, the same as set forth above in connection with the method according to the invention applies.

In another preferred embodiment, the organism, preferably microorganism, according to the invention is a genetically modified organism/microorganism derived from an organism/microorganism which naturally does not produce the respective alcohol but which has been genetically modified so as to produce said alcohol, i.e. by introducing the gene(s) necessary for allowing the production of the alcohol in the organism/microorganism. In principle any organism/microorganism can be genetically modified in this way. The enzymes responsible for the synthesis of the respective alcohols are generally known. Genes encoding corresponding enzymes are known in the art and can be used to genetically modify a given organism, preferably microorganism so as to produce the alcohol.

In a further preferred embodiment the organism, preferably a microorganism, according to the invention is genetically modified so as to express a hydratase enzyme which is capable of catalyzing the conversion of an alcohol into an alkene. In this context, the term "recombinant" means in a first aspect that the organism contains a foreign nucleic acid molecule encoding a corresponding hydratase enzyme. The term "foreign" in this context means that the nucleic acid molecule does not naturally occur in said organism/microorganism. This means that it does not occur in the same structure or at the same location in the organism/microorganism. In one preferred embodiment, the foreign nucleic acid molecule is a recombinant molecule comprising a promoter and a coding sequence encoding said hydratase enzyme in which the promoter driving expression of the coding sequence is heterologous with respect to the coding sequence. Heterologous in this context means that the promoter is not the promoter naturally driving the expression of said coding sequence but is a promoter naturally driving expression of a different coding sequence, i.e., it is derived from another gene, or is a synthetic promoter or a chimeric promoter. Preferably, the promoter is a promoter heterologous to the organism/microorganism, i.e. a promoter which does naturally not occur in the respective organism/microorganism. Even more preferably, the promoter is an inducible promoter. Promoters for driving expression in different types of organisms, in particular microorganisms, are well known to the person skilled in the art.
In another preferred embodiment the nucleic acid molecule is foreign to the organism/microorganism in that the encoded hydratase enzyme is not endogenous to the organism/microorganism, i.e. is naturally not expressed by the organism/microorganism when it is not genetically modified. In other words, the encoded hydratase enzyme is heterologous with respect to the organism/microorganism.
The term "recombinant" in another aspect means that the organism is genetically modified in the regulatory region controlling the expression of an enzyme as defined above which naturally occurs in the organism so as to lead to an increase in expression of the respective enzyme in comparison to a corresponding non-genetically modified organism. The meaning of the term high "higher expression" is described further below.
Such a modification of a regulatory region can be achieved by methods known to the person skilled in the art. One example is to exchange the naturally occurring promoter by a promoter which allows for a higher expression or to modify the naturally occurring promoter so as to show a higher expression. Thus, in this embodiment the organism contains in the regulatory region of the gene encoding an enzyme as defined above a foreign nucleic acid molecule which naturally does not occur in the organism and which leads to a higher expression of the enzyme in comparison to a corresponding non-genetically modified organism.

In a further preferred embodiment the organism/microorganism is characterized in that the expression/activity of the hydratase enzyme is higher in the organism/microorganism genetically modified with the foreign nucleic acid molecule in comparison to the corresponding non-genetically modified organism/microorganism. A "higher" expression/activity means that the expression/activity of the hydratase enzyme in the genetically modified organism/microorganism is at least 10%, preferably at least 20%, more preferably at least 30% or 50%, even more preferably at least 70% or 80% and particularly preferred at least 90% or 100% higher than in the corresponding non-genetically modified organism/microorganism. In even more preferred embodiments the increase in expression/activity may be at least 150%, at least 200% or at least 500%. In particularly preferred embodiments the expression is at least 10-fold, more preferably at least 100-fold and even more preferred at least 1000-fold higher than in the corresponding non-genetically modified organism/microorganism.
The term "higher" expression/activity also covers the situation in which the corresponding non-genetically modified organism/microorganism does not express a corresponding hydratase enzyme so that the corresponding expression/activity in the non-genetically modified organism/microorganism is zero.
Methods for measuring the level of expression of a given protein in a cell are well known to the person skilled in the art. In one embodiment, the measurement of the level of expression is done by measuring the amount of the corresponding protein. Corresponding methods are well known to the person skilled in the art and include Western Blot, ELISA etc. In another embodiment the measurement of the level of expression is done by measuring the amount of the corresponding RNA. Corresponding methods are well known to the person skilled in the art and include, e.g., Northern Blot.
Methods for measuring the enzymatic activity of the hydratase enzyme are known in the art and have already been described above.

The term "organism" as used in the context of the present invention refers in general to any possible type of organism, in particular eukaryotic organisms, prokaryotic organisms and archaebacteria. The term includes animal, plants, fungi, bacteria and archaebacteria. The term also includes isolated cells or cell aggregates of such organisms, like tissue or calli.
In one preferred embodiment, the organism is a microorganism. The term "microorganism" in the context of the present invention refers to prokaryotic cells, in particular bacteria, as well as to fungi, such as yeasts, and also to algae and archaebacteria. In one preferred embodiment, the microorganism is a bacterium. In principle any bacterium can be used. Preferred bacteria to be employed in the process according to the invention are bacteria of the genus Bacillus, Clostridium, Pseudomonas, Zymomonas or Escherichia. In a particularly preferred embodiment the bacterium belongs to the genus Escherichia and even more preferred to the species Escherichia coli.
In another preferred embodiment the microorganism is a fungus, more preferably a fungus of the genus Saccharomyces, Schizosaccharomyces, Aspergillus or Trichoderma and even more preferably of the species Saccharomyces cerevisiae, Schizosaccharomyces pombe, Aspergillus niger or of the species Trichoderma reesei.

In still another preferred embodiment the microorganism is a photosynthetically active microorganism such as bacteria which are capable of carrying out photosynthesis or micro-algae.
In a particularly preferred embodiment the microorganism is an algae, more preferably an algae from the genus belonging to the diatomeae.

In another preferred embodiment the organism according to the invention is an organism which is capable of photosynthesis, such as a plant or micro-algae. In principle, it can be any possible plant, i.e. a monocotyledonous plant or a dicotyledonous plant. It is preferably a plant which can be cultivated on an agriculturally meaningful scale and which allows to produce large amounts of biomass. Examples are grasses like Lolium, cereals like rye, barley, oat, millet, maize, other starch storing plants like potato or sugar storing plants like sugar cane or sugar beet. Conceivable is also the use of tobacco or of vegetable plants such as tomato, pepper, cucumber, egg plant etc. In another preferred embodiment the plant is an oil storing plants such as rape seed, olives etc. Also conceivable is the use of trees, in particular fast growing trees such as eucalyptus, poplar or rubber tree (Hevea brasiliensis).

In a particularly preferred embodiment the organism/microorganism employed in the method according to the invention is an organism/microorganism which is thermophilic in the sense that it can survive and catalyze the dehydration of the alcohol in question at elevated temperatures. The term "elevated" temperature means a temperature over 37°C. Examples for such organism/microorganism are bacteria of the genus Thermus, e.g. Thermus thermophilus or Thermus aquaticus, or bacteria of the genus Clostridium, such as Clostridium thermocellum. Other examples are microorganisms which are extremely heat-tolerant, e.g. microorganisms of the genus Thermotoga, such as Thermotoga maritime, or microorganisms of the genus Aquifex, such as Aquifex aeolicus.

The present invention also relates to the use of an organism, preferably a microorganism, which is characterized by the following features:
(a) it is capable of producing an alcohol; and
(b) it expresses a hydratase enzyme which is capable of catalyzing the conversion of said alcohol into an alkene
for the production of an alkene.
I.e., the present invention also relates to the use of an organism/microorganism according to the invention for the production of an alkene from the respective alcohol.

The present invention also relates to a composition comprising an organism according to the present invention.

Moreover, the present invention also relates to a composition comprising (i) an alcohol; and (ii) a hydratase enzyme which is capable of catalyzing the conversion of said alcohol into an alkene.
For the preferred embodiments of the enzyme the same applies as has already been set forth above in connection with the method and the organism according to the invention.

Moreover, the present invention also relates to the use of a hydratase enzyme which is capable of catalyzing the conversion of an alcohol into an alkene for the production of an alkene as defined herein-above from said alcohol.
For the preferred embodiments of the enzyme the same applies as has already been set forth above in connection with the method and the organism according to the invention.

Finally, the present invention also relates to the use of an alcohol for the production of an alkene, comprising the enzymatic conversion of the alcohol by a hydratase enzyme which is capable of catalyzing the conversion of the alcohol into an alkene. In a preferred embodiment the enzymatic conversion is achieved by an enzyme as described above in connection with the method according to the invention, more preferably with an oleate hydratase and most preferably the conversion is achieved by the use of an organism according to the invention.
- **Figure 1:**: shows a scheme of the reaction catalyzed by oleate hydratase and the generic reaction of converting an alcohol into an alkene.

The following Examples serve to illustrate the invention.

### Example 1: Cloning, expression and purification of a collection of oleate hydratases

A library of genes encoding enzymes homologous to the oleate hydratase (EC4.2.1.53) of *Elizabethkingia meningoseptica* (accession number GQ1444652) is cloned, expressed and purified.

### Cloning, bacterial cultures and expression of proteins

Amino-acid sequences corresponding to proteins homologous to the oleate hydratase from *Elizabethkingia meningoseptica* (accession number GQ1444652) are obtained from sequence database using the BLAST algorithm. A set of 20 sequences are chosen for maximizing genetic distance. In order to obtain a high level of expression in *E. coli,* the DNA sequences inferred from these protein sequences are generated taking in consideration the Codon Adaptation Index, as defined by Sharp and Li (Nucl. Acids Res. 15 (1987), 1281-1295).
The optimized sequences are synthesized by oligonucleotide concatenation (Geneart) and cloned in a pET25 expression vector, with the addition of a methionine initiation codon and a 6-histidine tag added at the N-terminal end immediately preceding the first methionine of the original protein sequence. Competent *E. coli* BL21(DE3) cells (Novagen) are transformed with these vectors by heat shock. The cells are grown with shaking (160 rpm) at 30°C in TB medium containing 0.5 M sorbitol, 5 mM betain, 100 µg/ml ampicillin until reaching an OD at 600 nm comprised between 0.8 and 1. Isopropyl-B-D-thiogalactopyranoside (IPTG) is then added to a final concentration of 1 mM and protein expression is continued at 20°C overnight (approximately 16 h). The cells are collected by centrifugation at 4°C, 10.000 rpm for 20 min and the pellets are frozen at -80°C.

### Cell lysis

1.6 g of cells are thawed on ice and resuspended in 5 ml of 50 mM Na₂HPO₄ pH 8 containing 300 mM NaCl, 5 mM MgCl₂, 1 mM DTT. Twenty microliters of lysonase (Novagen) are added. Cells are incubated for 10 min at room temperature and then returned to ice for 20 min. Cell lysis is completed by sonication for 3x 5 min in an ultrasound water bath at 0°C; samples are homogenized between pulses. The bacterial extracts are then clarified by centrifugation at 4°C, 10,000 rpm for 20 min.

### Protein purification and concentration (PROTlNO kit)

The clarified bacterial lysates are loaded on a PROTINO-1000 Ni-IDA column (Macherey-Nagel) allowing adsorption of 6-His-tagged proteins. Columns are washed and the enzymes of interest are eluted with 4 ml of 50 mM Na₂HPO₄ pH 8 containing 300 mM NaCl, 5 mM MgCl₂, 1 mM DTT, 250 mM imidazole. Eluates are then concentrated in Amicon Ultra-4 10 kDa cells (Millipore) to a final volume of 250 µl. Proteins are quantified by the Bradford method. SDS-PAGE analyses are conducted in parallel: a main band is clearly visible around 65kD, and the purity of the protein is estimated to over 80%.

### Example 2: Measuring the activity of purified oleate hydratases towards ethanol

### Enzymatic reactions

Reactions are run in 1 ml of buffer containing 20mM Tris (pH 8) and 50 mM NaCl, and various concentration of ethanol, ranging from 100mM to 5M. 1 mg of enzyme is added to the sample. The enzyme-free control reactions are carried out in parallel. The 1 ml reactions are placed in 2 ml vials (Interchim) and sealed with a teflon/silica/teflon septum (Interchim). Reactions are incubated without shaking at 37°C for 72 h.

### Analysis of reactions

The gas present in the headspace is collected using a gas syringe equipped with an anti-backup mechanism. The gas sample is analyzed by gas chromatography (GC) coupled with a flame ionization detector.
Column: BPX5 (SGE)
GC: Hewlett Packard 5890 series II
For each chromatogram, the presence of a peak is analyzed precisely, and compared to negative control chromatograms and chromatograms obtained when injecting pure ethylene (Sigma). A significant production of a gas corresponding to the elution time of ethylene is observed in the case of several enzymatic preparations.

The identity of the gas if further confirmed using GC/MS analysis, giving the precise physical signature of ethylene.
Column: BPX5 (SGE)
GC/MS: MSD 5973 (HP)

### Example 3: Measuring the activity of purified oleate hydratases towards propanol isomers

The same protocol as in Example 2 is followed to assay the activity of the collection of 20 enzymes towards propan-1-ol and propan-2-ol (isopropanol) instead of ethanol. A signal indicating the significant production of a gas having the same elution time than propylene is identified in the case of several enzymes.
The identity of propylene is further confirmed in GC/MS analyses.

### Example 4: Measuring the activity of purified oleate hydratases towards linear butanol isomers

The same protocol as in Example 2 is followed to assay the activity of the collection of 20 enzymes towards butan-1-ol (*n*-butanol), butan-2-ol (*sec*-butanol) instead of ethanol. A signal indicating the significant production of a gas having the same elution time than linear butene (but-1-ene or the cis or trans isomers of but-2-ene) is identified in the case of several enzymes.
The identity of the gas is further confirmed in GC/MS analyses. In the case of several enzymes, only one of the three linear butenes is obtained. In some other cases, a mix of two or the three compounds is obtained.

### Example 5: Measuring the activity of purified oleate hydratases towards branched butanol isomers

The same protocol as in Example 2 is followed to assay the activity of the collection of 20 enzymes towards 2-methylpropan-1-ol (isobutanol) or 2-methylpropan-2-ol (*tert*-butanol) instead of ethanol. A signal indicating the significant production of a gas having the same elution time than 2-methylprop-1-ene (isobutene) is identified in the case of several enzymes.
The identity of the gases is further confirmed in GC/MS analyses.

### Example 6: Measuring the activity of purified oleate hydratases towards isopentenol

The same protocol as in Example 2 is followed to assay the activity of the collection of 20 enzymes towards isopentenol isomers (3-methylbut-3-en-1-ol, 3-methylbut-3-en-2-ol, 2-methylbut-3-en-1-ol, 2-methylbut-3-en-2-ol) instead of ethanol. A signal indicating the significant production of a gas having the same elution time than 2-methylbuta-1,3-diene (isoprene) is identified in the case of several enzymes.
The identity of isoprene is further confirmed in GC/MS analyses.

### Example 7: Measuring the activity of purified oleate hydratases towards branched butenol isomers

The same protocol as in Example 2 is followed to assay the activity of the collection of 20 enzymes towards but-3-en-1-ol or but-3-en-2-ol instead of ethanol. A signal indicating the significant production of a gas having the same elution time than buta-1,3-diene is identified in the case of several enzymes.
The identity of buta-1,3-diene is further confirmed in GC/MS analyses.

### Example 8: Measuring the activity of purified oleate hydratases towards phenyl-ethanol isomers

The same protocol as in Example 2 is followed to assay the activity of the collection of 20 enzymes towards 1-phenylethanol or 2-phenylethanol instead of ethanol. As the expected product, vinylbenzene (styrene), is liquid at ambient temperature, what is harvested here is not gas from the headspace, but liquid from the reaction mix. Two microliter of such reaction mix are injected in the GC apparatus, and the signal indicating the significant production of a gas having the same elution time than styrene is identified in the case of several enzymes.
The identity of the gas is further confirmed in GC/MS analyses.

### Example 9: Optimization of reaction conditions by using a cofactor

The same assays as followed in Examples 2 to 8 are carried out, but a carboxylic acid is added as cofactor to the reaction mix.
Vials containing the buffer, an enzyme preparation, an alcohol and 1 mg/mL of a carboxylic acid are prepared and tested following the same protocol as described in Example 2.

Cofactors are used in accordance with the list presented in Table 3. In particular, linear octanoïc acid is used as cofactor in the case of ethanol, propan-2-ol, 2-methylpropan-2-ol.
An increase in the rate of conversion of the alcohol into the corresponding alkene is observed in several cases.

### Example 10: Bacteria synthesizing ethylene from glucose

Ethanol producing *E. coli* bacteria are transformed using a plasmid encoding an enzyme having shown activity in converting ethanol into ethylene, as described in Example 2. The transformed bacteria are then grown in mineral medium containing 20g/L glucose under aeration at 30°C during 24 hours. The production of ethylene in the headspace is measured by taking a sample using a gas syringe, and using a GC apparatus as described in Example 2.

### Example 11: Bacteria synthesizing propylene from glucose

*E. coli* bacteria engineered to produce propan-2-ol as described in Jojima et al. (2008, Appl. Microbial. Biotechnol. 77:1219-1224) are transformed by a plasmid encoding an enzyme of the oleate hydratase family showing activity in converting propan-2-ol into propylene, as described in Example 3. The transformed bacteria are then grown in mineral medium containing 20g/L glucose under aeration at 30°C during 24 hours. The production of propylene in the headspace is measured by taking a sample using a gas syringe, and using a GC apparatus as previously described.

### Example 12: Bacteria synthesizing isobutene from glucose

*E. coli* bacteria engineered to produce 2-methylpropan-1-ol (isobutanol), as described in Connor and Liao, Curr. Opin. Biotechnol. 20, 2009:307, are transformed using a plasmid encoding an enzyme having shown activity in converting branched butanol isomers into isobutene, as described in Example 5. The transformed bacteria are then grown in mineral medium containing 20g/L glucose under aeration at 30°C during 24 hours. The production of isobutene in the headspace is measured by taking a sample using a gas syringe, and using a GC apparatus as previously described.

### Example 13: Bacteria synthesizing isoprene from glucose

*E. coli* bacteria first engineered to produce 3-methylbut-3-en-1-ol are further transformed using a plasmid encoding an enzyme having shown activity in converting branched methyl-butenol isomers into isoprene, as described in Example 6. The transformed bacteria are then incubated in a fermentor using 50g/l sucrose, and stirred under oxygenation at 30°C during 24 hours. The production of isoprene in the headspace is measured by taking a sample using a gas syringe, and using a GC apparatus as previously described.

### Example 14: Bacteria synthesizing butadiene from glucose

*E. coli* bacteria first engineered to produce but-3-en-1-ol are transformed using a plasmid encoding an enzyme having shown activity in converting butenol isomers into butadiene, as described in Example 7. The transformed bacteria are then grown in mineral medium containing 20g/L glucose under aeration at 30°C during 24 hours. The production of butadiene in the headspace is measured by taking a sample using a gas syringe, and using a GC apparatus as previously described.

### Example 15: Bacteria synthesizing styrene from glucose

*E. coli* bacteria first engineered to produce 1-phenylethanol are transformed using a plasmid encoding an enzyme having shown activity in converting branched butenol isomers into vinylbenzene (styrene), as described in Example 8. The transformed bacteria are then grown in mineral medium containing 20g/L glucose under aeration at 30°C during 24 hours. Two microliters of the liquid fraction of the reaction mix is harvested using a syringe, and injected in a GC apparatus as previously described to identify the presence of styrene.

## Claims

1. A method for producing an alkene, the method comprising a step of converting an alcohol by an enzymatic dehydration step into an alkene, wherein:
the alcohol is a compound of formula (I) 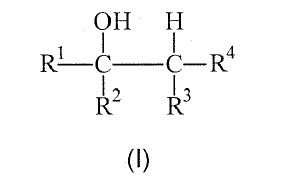
wherein R¹, R², R³ and R⁴ are each independently selected from hydrogen or C₁₋₁₁ hydrocarbyl; and
the alkene is a compound of formula (II) 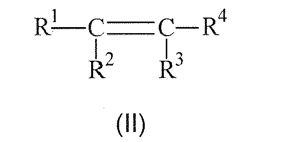
wherein R¹, R², R³ and R⁴ have the same meanings as defined for the compound of formula (I).

2. The method of claim 1 wherein the enzymatic dehydration step is catalyzed by a hydratase.

3. The method of claim 1 or 2 wherein the enzymatic dehydration step is catalyzed by an unsaturated fatty acid hydratase.

4. The method of claim 3 wherein the fatty acid hydratase is an oleate hydratase (EC 4.2.1.53).

5. The method of claim 4 wherein the oleate hydratase has an amino acid sequence as set forth in any one of SEQ ID NOs: 1 to 11 or a sequence having at least 15% sequence identity to any one of SEQ ID NO: 1 to 11 preferably 30%, 40%, 50%, 60%, 80% or 90%.

6. The method of any one of claims 1 to 5wherein:
(i) the alcohol is ethanol and the alkene is ethene; or
(ii) the alcohol is propan-1-ol and the alkene is propene; or
(iii) the alcohol is propan-2-ol and the alkene is propene; or
(iv) the alcohol is butan-1-ol and the alkene is but-1-ene; or
(v) the alcohol is 2-methylpropan-1-ol and the alkene is 2-methylprop-1-ene; or
(vi) the alcohol is 2-methylpropan-2-ol and the alkene is 2-methylprop-1-ene; or
(vii) the alcohol is butan-2-ol and the alkene is but-1-ene; or
(viii) the alcohol is butan-2-ol and the alkene is but-2-ene; or
(ix) the alcohol is 3-methylbut-3-en-1-ol and the alkene is 2-methylbuta-1,3-diene; or
(x) the alcohol is 2-methylbut-3-en-2-ol and the alkene is 2-methylbuta-1,3-diene; or
(xi) the alcohol is 2-methylbut-3-en-1-ol and the alkene is 2-methylbuta-1,3-diene; or
(xii) the alcohol is 3-methylbut-3-en-2-ol and the alkene is 2-methylbuta-1,3-diene; or
(xiii) the alcohol is but-3-en-1-ol and the alkene is buta-1,3-diene; or
(xiv) the alcohol is but-3-en-2-ol and the alkene is buta-1,3-diene; or
(xv) the alcohol is 1-phenylethanol and the alkene is styrene; or
(xvi) the alcohol is 2-phenylethanol and the alkene is styrene.

7. The method of any one of claims 1 to 6 wherein the step of converting an alcohol by an enzymatic dehydration step into an alkene is carried out at a temperature above 37°C and the enzymatic dehydration step is catalyzed by a thermophilic hydratase.

8. The method of claim 7 wherein the alkene comprises 6 or 7 carbon atoms.

9. The method of any one of claims 1 to 8 wherein the step of converting an alcohol by an enzymatic dehydration step into an alkene is realized in the presence of an organism capable of expressing an enzyme as defined in any one of claims 2 to 5.

10. The method of claim 19 wherein the microorganism is furthermore capable of producing the alcohol to be converted.

11. A recombinant organism which is **characterized by** the following features:
(a) it is capable of producing an alcohol as defined in claim 1; and
(b) it expresses a hydratase enzyme which is capable of catalyzing the conversion of said alcohol into an alkene.

12. Use of an organism as defined in claim 11 for the production of an alkene.

13. A composition comprising an organism of claim 11.

14. A composition comprising
(i) an alcohol as defined in claim 1; and
(ii) a hydratase enzyme which is capable of catalyzing the conversion of said alcohol into an alkene.

15. Use of an enzyme as defined in any one of claims 2 to 5 for the production of an alkene.


SEQUENCE LISTING

<110>  Marliere , Philippe

<120>  Method for producing an alkene comprising the step of converting
       an alcohol by an enzymatic dehydration step

<130>  R3093 EP S3

<160>  11

<170>  PatentIn version 3.5

<210>  1
<211>  590
<212>  PRT
<213>  Streptococcus pyogenes

<400>  1

Met Tyr Tyr Thr Ser Gly Asn Tyr Glu Ala Phe Ala Thr Pro Arg Lys
1               5                   10                  15

Pro Glu Gly Val Asp Gln Lys Ser Ala Tyr Ile Val Gly Thr Gly Leu
            20                  25                  30

Ala Gly Leu Ala Ala Ala Val Phe Leu Ile Arg Asp Gly His Met Ala
        35                  40                  45

Gly Glu Arg Ile His Leu Phe Glu Glu Leu Pro Leu Ala Gly Gly Ser
    50                  55                  60

Leu Asp Gly Ile Glu Lys Pro His Leu Gly Phe Val Thr Arg Gly Gly
65                  70                  75                  80

Arg Glu Met Glu Asn His Phe Glu Cys Met Trp Asp Met Tyr Arg Ser
                85                  90                  95

Ile Pro Ser Leu Glu Ile Pro Gly Ala Ser Tyr Leu Asp Glu Phe Tyr
            100                 105                 110

Trp Leu Asp Lys Asp Asp Pro Asn Ser Ser Asn Cys Arg Leu Ile His
        115                 120                 125

Lys Arg Gly Asn Arg Val Asp Asp Asp Gly Gln Tyr Thr Leu Gly Lys
    130                 135                 140

Gln Ser Lys Glu Leu Val His Leu Ile Met Lys Thr Glu Glu Ser Leu
145                 150                 155                 160

Gly Asp Gln Thr Ile Glu Glu Phe Phe Ser Glu Asp Phe Phe Lys Ser
                165                 170                 175

Asn Phe Trp Ile Tyr Trp Ala Thr Met Phe Ala Phe Glu Lys Trp Gln
            180                 185                 190

Phe Cys Cys Arg Asn Ala Gly Tyr Ala Met Arg Phe Ile His His Ile
        195                 200                 205

Asp Gly Leu Pro Asp Phe Thr Ser Leu Lys Phe Asn Lys Tyr Asn Gln
    210                 215                 220

Tyr Asp Ser Met Val Lys Pro Ile Ile Ala Tyr Leu Glu Ser His Asp
225                 230                 235                 240

Val Asp Ile Gln Phe Asp Thr Lys Val Thr Asp Ile Gln Val Glu Gln
                245                 250                 255

Thr Ala Gly Lys Lys Val Ala Lys Thr Ile His Met Thr Val Ser Gly
            260                 265                 270

Glu Ala Lys Ala Ile Glu Leu Thr Pro Asp Asp Leu Val Phe Val Thr
        275                 280                 285

Asn Gly Ser Ile Thr Glu Ser Ser Thr Tyr Gly Ser His His Glu Val
    290                 295                 300

Ala Lys Pro Thr Lys Ala Leu Gly Gly Ser Trp Asn Leu Trp Glu Asn
305                 310                 315                 320

Leu Ala Ala Gln Ser Asp Asp Phe Gly His Pro Lys Val Phe Tyr Gln
                325                 330                 335

Asp Leu Pro Ala Glu Ser Trp Phe Val Ser Ala Thr Ala Thr Ile Lys
            340                 345                 350

His Pro Ala Ile Glu Pro Tyr Ile Glu Arg Leu Thr His Arg Asp Leu
        355                 360                 365

His Asp Gly Lys Val Asn Thr Gly Gly Ile Ile Thr Ile Thr Asp Ser
    370                 375                 380

Asn Trp Met Met Ser Phe Ala Ile His Arg Gln Pro His Phe Lys Glu
385                 390                 395                 400

Gln Lys Asp Asn Glu Thr Thr Val Trp Ile Tyr Gly Leu Tyr Ser Asn
                405                 410                 415

Ser Glu Gly Asn Tyr Val His Lys Lys Ile Glu Glu Cys Thr Gly Gln
            420                 425                 430

Glu Ile Thr Glu Glu Trp Leu Tyr His Leu Gly Val Pro Val Asp Lys
        435                 440                 445

Ile Lys Asp Leu Ala Ser Gln Asp Tyr Ile Asn Thr Val Pro Val Tyr
    450                 455                 460

Met Pro Tyr Ile Thr Ser Tyr Phe Met Pro Arg Val Lys Gly Asp Arg
465                 470                 475                 480

Pro Lys Val Ile Pro Asp Gly Ser Val Asn Leu Ala Phe Ile Gly Asn
                485                 490                 495

Phe Ala Glu Ser Pro Ser Arg Asp Thr Val Phe Thr Thr Glu Tyr Ser
            500                 505                 510

Ile Arg Thr Ala Met Glu Ala Val Tyr Ser Phe Leu Asn Val Glu Arg
        515                 520                 525

Gly Ile Pro Glu Val Phe Asn Ser Ala Tyr Asp Ile Arg Glu Leu Leu
    530                 535                 540

Lys Ala Phe Tyr Tyr Leu Asn Asp Lys Lys Ala Ile Lys Asp Met Asp
545                 550                 555                 560

Leu Pro Ile Pro Ala Leu Ile Glu Lys Ile Gly His Lys Lys Ile Lys
                565                 570                 575

Asp Thr Phe Ile Glu Glu Leu Leu Lys Asp Ala Asn Leu Met
            580                 585                 590

<210>  2
<211>  646
<212>  PRT
<213>  Elizabethkingia meningoseptica

<400>  2

Met Asn Pro Ile Thr Ser Lys Phe Asp Lys Val Leu Asn Ala Ser Ser
1               5                   10                  15

Glu Tyr Gly His Val Asn His Glu Pro Asp Ser Ser Lys Glu Gln Gln
            20                  25                  30

Arg Asn Thr Pro Gln Lys Ser Met Pro Phe Ser Asp Gln Ile Gly Asn
        35                  40                  45

Tyr Gln Arg Asn Lys Gly Ile Pro Val Gln Ser Tyr Asp Asn Ser Lys
    50                  55                  60

Ile Tyr Ile Ile Gly Ser Gly Ile Ala Gly Met Ser Ala Ala Tyr Tyr
65                  70                  75                  80

Phe Ile Arg Asp Gly His Val Pro Ala Lys Asn Ile Thr Phe Leu Glu
                85                  90                  95

Gln Leu His Ile Asp Gly Gly Ser Leu Asp Gly Ala Gly Asn Pro Thr
            100                 105                 110

Asp Gly Tyr Ile Ile Arg Gly Gly Arg Glu Met Asp Met Thr Tyr Glu
        115                 120                 125

Asn Leu Trp Asp Met Phe Gln Asp Ile Pro Ala Leu Glu Met Pro Ala
    130                 135                 140

Pro Tyr Ser Val Leu Asp Glu Tyr Arg Leu Ile Asn Asp Asn Asp Ser
145                 150                 155                 160

Asn Tyr Ser Lys Ala Arg Leu Ile Asn Asn Lys Gly Glu Ile Lys Asp
                165                 170                 175

Phe Ser Lys Phe Gly Leu Asn Lys Met Asp Gln Leu Ala Ile Ile Arg
            180                 185                 190

Leu Leu Leu Lys Asn Lys Glu Glu Leu Asp Asp Leu Thr Ile Glu Asp
        195                 200                 205

Tyr Phe Ser Glu Ser Phe Leu Lys Ser Asn Phe Trp Thr Phe Trp Arg
    210                 215                 220

Thr Met Phe Ala Phe Glu Asn Trp His Ser Leu Leu Glu Leu Lys Leu
225                 230                 235                 240

Tyr Met His Arg Phe Leu His Ala Ile Asp Gly Leu Asn Asp Leu Ser
                245                 250                 255

Ser Leu Val Phe Pro Lys Tyr Asn Gln Tyr Asp Thr Phe Val Thr Pro
            260                 265                 270

Leu Arg Lys Phe Leu Gln Glu Lys Gly Val Asn Ile His Leu Asn Thr
        275                 280                 285

Leu Val Lys Asp Leu Asp Ile His Ile Asn Thr Glu Gly Lys Val Val
    290                 295                 300

Glu Gly Ile Ile Thr Glu Gln Asp Gly Lys Glu Val Lys Ile Pro Val

305                 310                 315                 320

Gly Lys Asn Asp Tyr Val Ile Val Thr Thr Gly Ser Met Thr Glu Asp
                325                 330                 335

Thr Phe Tyr Gly Asn Asn Lys Thr Ala Pro Ile Ile Gly Ile Asp Asn
            340                 345                 350

Ser Thr Ser Gly Gln Ser Ala Gly Trp Lys Leu Trp Lys Asn Leu Ala
        355                 360                 365

Ala Lys Ser Glu Ile Phe Gly Lys Pro Glu Lys Phe Cys Ser Asn Ile
    370                 375                 380

Glu Lys Ser Ala Trp Glu Ser Ala Thr Leu Thr Cys Lys Pro Ser Ala
385                 390                 395                 400

Leu Ile Asp Lys Leu Lys Glu Tyr Ser Val Asn Asp Pro Tyr Ser Gly
                405                 410                 415

Lys Thr Val Thr Gly Gly Ile Ile Thr Ile Thr Asp Ser Asn Trp Leu
            420                 425                 430

Met Ser Phe Thr Cys Asn Arg Gln Pro His Phe Pro Glu Gln Pro Asp
        435                 440                 445

Asp Val Leu Val Leu Trp Val Tyr Ala Leu Phe Met Asp Lys Glu Gly
    450                 455                 460

Asn Tyr Ile Lys Lys Thr Met Leu Glu Cys Thr Gly Asp Glu Ile Leu
465                 470                 475                 480

Ala Glu Leu Cys Tyr His Leu Gly Ile Glu Asp Gln Leu Glu Asn Val
                485                 490                 495

Gln Lys Asn Thr Ile Val Arg Thr Ala Phe Met Pro Tyr Ile Thr Ser
            500                 505                 510

Met Phe Met Pro Arg Ala Lys Gly Asp Arg Pro Arg Val Val Pro Glu
        515                 520                 525

Gly Cys Lys Asn Leu Gly Leu Val Gly Gln Phe Val Glu Thr Asn Asn
    530                 535                 540

Asp Val Val Phe Thr Met Glu Ser Ser Val Arg Thr Ala Arg Ile Ala
545                 550                 555                 560

Val Tyr Lys Leu Leu Asn Leu Asn Lys Gln Val Pro Asp Ile Asn Pro
                565                 570                 575

Leu Gln Tyr Asp Ile Arg His Leu Leu Lys Ala Ala Lys Thr Leu Asn
            580                 585                 590

Asp Asp Lys Pro Phe Val Gly Glu Gly Leu Leu Arg Lys Val Leu Lys
        595                 600                 605

Gly Thr Tyr Phe Glu His Val Leu Pro Ala Gly Ala Ala Glu Glu Glu
    610                 615                 620

Glu His Glu Ser Phe Ile Ala Glu His Val Asn Lys Phe Arg Glu Trp
625                 630                 635                 640

Val Lys Gly Ile Arg Gly
                645

<210> 3
<211> 625
<212> PRT
<213> Bifidobacterium longum

<400> 3

Met Tyr Tyr Ser Ser Gly Asn Tyr Glu Ala Phe Ala Arg Pro Lys Lys
1               5                   10                  15

Pro Ala Asp Val Asp Asn Lys His Ala Tyr Ile Ile Gly Thr Gly Leu
            20                  25                  30

Ala Ala Leu Ser Ala Ala Cys Tyr Leu Val Arg Asp Gly Gln Met Pro
        35                  40                  45

Gly Glu Asn Ile His Ile Leu Glu Lys Asp Pro Val Pro Gly Gly Ala
    50                  55                  60

Cys Asp Gly Leu Asp Ile Pro Gly Leu Gly Tyr Val Met Arg Gly Gly
65                  70                  75                  80

Arg Glu Met Asp Asn His Phe Glu Val Met Trp Asp Leu Phe Arg Ser
                85                  90                  95

Ile Pro Ser Ile Glu Thr Glu Gly Val Ser Val Leu Asp Glu Tyr Tyr
            100                 105                 110

Trp Leu Asn Lys Glu Asp Pro Asn Tyr Ser Leu Cys Arg Ala Thr Lys
        115                 120                 125

Asn Leu Gly Lys Asp Ala Gly Leu Lys Gly Lys Phe Gly Leu Ser Asp
    130                 135                 140

Lys Ala Ser Met Glu Ile Met Lys Leu Phe Phe Thr Pro Asp Glu Asp
145                 150                 155                 160

Leu Tyr Asp Lys Pro Ile Thr Asp Phe Phe Asp Asp Glu Val Leu Asn
                165                 170                 175

Ser Asn Phe Trp Leu Tyr Trp Arg Thr Met Phe Ala Phe Glu Asn Trp
            180                 185                 190

His Ser Ala Leu Glu Met Lys Leu Tyr Ile Lys Arg Tyr Ile His His
        195                 200                 205

Ile Gly Gly Leu Pro Asp Phe Ser Ala Leu Arg Phe Thr Arg Tyr Asn
    210                 215                 220

Gln Tyr Glu Ser Met Ile Leu Pro Met Val Lys Tyr Leu Glu Ser His
225                 230                 235                 240

Gly Val Glu Phe Arg Tyr Asn Thr Lys Val Glu Asn Val Glu Phe Ala
                245                 250                 255

Ile Gly Gly Gly Ala Gly Pro Lys Arg Glu His Thr Gly Val Gly Gln
            260                 265                 270

Asp Thr Ile Gln Lys Ile Gln Ala Thr Ser Gly Phe Phe Lys Arg Asn
        275                 280                 285

Pro Ala Ser Thr Pro Thr Lys Lys Leu Ala Val Arg Ile Asp Val Asp
    290                 295                 300

His Glu Gly Asp Lys Ser Ser Ile Asp Leu Thr Glu Asn Asp Leu Val
305                 310                 315                 320

Phe Ile Thr Asn Gly Gly Cys Val Glu Asn Ser Thr Met Gly Ser Gln
                325                 330                 335

Asn Ser Pro Ala Ala Trp Asn Pro Asp Leu Lys Pro Gly Gly Gly Trp
            340                 345                 350

Asp Met Trp Lys Arg Ile Ala Asp Gln Asp Pro Ser Phe Gly His Pro
        355                 360                 365

Glu Lys Phe Cys Ser Asp Pro Asn Ala Thr Lys Trp Met Ser Ala Thr
    370                 375                 380

Val Thr Thr Leu Asp Gly Glu Ile Pro Pro Tyr Ile Gln Lys Ile Cys
385                 390                 395                 400

Lys Arg Asp Pro Phe Ser Gly Lys Val Val Thr Gly Gly Ile Val Thr
                405                 410                 415

Val Gln Asp Ser Asn Trp Leu Met Ser Trp Thr Leu Asn Arg Gln Gln
            420                 425                 430

Gln Phe Arg Asp Gln Pro Lys Asp Gln Leu Cys Val Trp Val Tyr Gly
        435                 440                 445

Leu Phe Pro Asp Lys Pro Gly Asn Tyr Val Lys Lys Pro Met Thr Glu
    450                 455                 460

Cys Thr Gly Glu Glu Ile Cys Glu Glu Trp Leu Tyr His Met Gly Val
465                 470                 475                 480

Pro Thr Asp Lys Ile Glu Ala Leu Ala Lys His His Ala Asn Thr Val
                485                 490                 495

Pro Val Met Met Pro Tyr Ile Thr Ala Phe Phe Met Pro Arg Ala Ala
            500                 505                 510

Gly Asp Arg Pro Asp Val Val Pro Asp Gly Ala Val Asn Phe Ala Phe
        515                 520                 525

Leu Gly Gln Phe Ala Glu Thr Pro Arg Asp Thr Ile Phe Thr Thr Glu
    530                 535                 540

Tyr Ser Met Arg Thr Gly Met Glu Ala Val Tyr Thr Leu Leu Gly Val
545                 550                 555                 560

Asp Arg Gly Val Pro Glu Val Trp Gly Ser Val Tyr Asp Val Arg Asn
                565                 570                 575

Leu Leu Asn Ala Thr Val Lys Leu Arg Asp Gly Ala Pro Val Thr Asp
            580                 585                 590

Met Lys Leu Asn Phe Ile Glu Lys Ala Val Val Lys Lys Val Leu Lys
        595                 600                 605

Lys Leu Asp Gly Thr Asp Ile Ala Thr Leu Leu Arg Glu Tyr Arg Val
    610                 615                 620

Ile
625

<210>  4
<211>  565
<212>  PRT
<213>  Clostridium botulinum

<400>  4

Met Gly Asn Tyr Gln Arg Asn Asn Thr Ile Pro Pro Gln Asn Ile Lys
1               5                   10                  15

Arg Gln Lys Ala Tyr Leu Val Gly Gly Gly Ile Ala Ser Leu Ala Ala
            20                  25                  30

Ala Phe Tyr Leu Ile Arg Asp Gly His Ile Asp Gly Lys Asn Ile Thr
        35                  40                  45

Ile Phe Glu Glu Leu Asn Ile Glu Gly Gly Ala Met Asp Gly Ala Gly
    50                  55                  60

Asn Ala Glu Thr Gly Tyr Ile Ile Arg Gly Gly Arg Glu Met Glu Glu
65                  70                  75                  80

His Tyr Glu Cys Thr Trp Asp Leu Phe Ala Asp Ile Pro Ser Leu Glu
                85                  90                  95

Asn Tyr Gly Tyr Ser Val Leu Asp Glu Phe Lys Glu Leu Asn Asp Asn
            100                 105                 110

Asp Pro Asn Ile Ser Lys Cys Arg Leu Ile His Asn Cys Gly Gln Lys
        115                 120                 125

Lys Asn Ser Asp Ser Met Glu Leu Ser Pro Ala His Ala Lys Arg Leu
    130                 135                 140

Thr Glu Leu Phe Leu Ala Thr Glu Asp Ala Leu Gly Asn Thr Thr Val
145                 150                 155                 160

Glu Gln Phe Phe Asp Glu Ser Phe Leu Asp Thr Asn Phe Trp Tyr Leu
                165                 170                 175

Trp Arg Ser Met Phe Ala Phe Glu Asn Trp His Gly Val Val Glu Met
            180                 185                 190

Lys Arg Tyr Met His Arg Phe Ile His Leu Leu Pro Gly Phe Asn Lys
        195                 200                 205

Met Lys Gly Leu Val Phe Pro Lys Tyr Asn Gln Tyr Asp Ser Phe Ile
    210                 215                 220

Leu Pro Leu Ser Lys His Leu Glu Ser Lys Gly Val Lys Phe Gln Tyr
225                 230                 235                 240

Gly Thr Gln Val Ile Asp Leu Asp Ile Asp Ile Lys Asp Lys Glu Lys
                245                 250                 255

Thr Val Thr Gly Ile His Ile Ile His Asp Asn Lys Glu Gln Val Ile
            260                 265                 270

Asn Ile Arg Ser Thr Asp Ile Val Met Val Thr Asn Gly Ser Met Thr
        275                 280                 285

Glu Cys Ser Glu Tyr Ala Asp Asn Lys Thr Val Pro Lys Leu Arg Lys
    290                 295                 300

Asp Leu Gly Gly Val Trp Ser Leu Trp Lys Asn Ile Ser Glu Lys Asp
305                 310                 315                 320

Pro Ala Phe Gly His Pro Glu Val Phe Cys Gly Asp Val Asp Arg Ser
                325                 330                 335

Lys Trp Glu Ser Phe Thr Ile Thr Cys Lys Asp Ser Lys Leu Val Asp
            340                 345                 350

Lys Ile Arg Glu Leu Ser Gly Asn Glu Pro His Ser Gly Lys Thr Val
        355                 360                 365

Thr Gly Gly Ile Ile Thr Met Thr Asp Ser Asn Trp Leu Leu Ser Val
    370                 375                 380

Thr Cys Asn Arg Gln Pro His Phe Pro Asp Gln Pro Asp Asp Val Ile
385                 390                 395                 400

Val Leu Trp Ala Tyr Gly Leu Leu Leu Asp Asn Glu Gly Asn Tyr Ile
                405                 410                 415

Lys Lys Lys Met Thr Glu Cys Thr Gly Glu Glu Leu Leu Lys Glu Leu
            420                 425                 430

Leu Tyr His Leu Gly Leu Glu Lys Asp Ile Pro Glu Ile Leu Lys Thr
        435                 440                 445

Asp Lys Val Ile Pro Ala Met Met Pro Tyr Ile Thr Ala Gln Phe Met
    450                 455                 460

Pro Arg Val Lys Gly Asp Arg Pro Asn Val Val Pro Glu Gly Ser Lys

465                 470                 475                 480

Asn Leu Ala Phe Leu Gly Gln Phe Ala Glu Val Pro Gly Asp Cys Val
                485                 490                 495

Phe Thr Val Glu Tyr Ser Ile Arg Ser Ala Ile Met Ala Thr Tyr Lys
            500                 505                 510

Leu Leu Asn Leu Asn Lys Lys Val Pro Glu Val His Pro Ser Gln Tyr
        515                 520                 525

Asp Ile Arg Val Ile Ala Asn Ala Val Lys Thr Leu Asn Ser Gly Arg
    530                 535                 540

Pro Leu Pro Ala Glu Thr Ile Ile Lys Lys Leu Leu Lys Asp Thr Ser
545                 550                 555                 560

Met Glu Gly Leu Ile
                565

<210>  5
<211>  666
<212>  PRT
<213>  Coprococcus eutactus ATCC 27759

<400>  5

Met Asp Ile Asp Gly Lys Leu Lys Gly Glu Leu Leu Met Ser Lys Lys
1               5                   10                  15

Lys Leu Gly Ile Gly Ile Ala Ala Val Ala Val Ala Gly Ala Gly Ala
            20                  25                  30

Ala Ala Leu Thr Val Lys Asn His Asn Lys Ala Lys Val Gln Lys Glu
        35                  40                  45

Lys Ala Ala Ala Glu His Ala Glu Tyr Arg Asn Thr Glu Arg Gly Lys
    50                  55                  60

His Glu Lys Asn Ser Lys Gly Ile Tyr Tyr Thr Asn Gly Asn Tyr Glu
65                  70                  75                  80

Ala Phe Ala Arg Pro Arg Lys Pro Glu Gly Val Asp Asp Lys Asn Ala
                85                  90                  95

Tyr Ile Val Gly Ser Gly Leu Ala Ser Leu Ala Ala Ala Cys Phe Leu
            100                 105                 110

Val Arg Asp Gly Gln Met Pro Gly Ser His Ile His Ile Leu Glu Ala

115                 120                 125

Met Asp Ile Ala Gly Gly Ala Cys Asp Gly Ile Phe Asp Pro Ala Arg
    130                 135                 140

Gly Tyr Val Met Arg Gly Gly Arg Glu Met Glu Asn His Phe Glu Cys
145                 150                 155                 160

Leu Trp Asp Leu Phe Arg Ser Ile Pro Ser Ile Glu Thr Pro Gly Val
                165                 170                 175

Ser Val Leu Asp Glu Tyr Tyr Trp Leu Asn Lys Glu Asp Pro Asn Tyr
            180                 185                 190

Ser Leu Cys Arg Ala Thr Val Asn Arg Gly Lys Asp Ala His Thr Asp
        195                 200                 205

Gly Lys Phe Asn Leu Ser Gln Lys Gly Cys Met Glu Ile Met Lys Leu
    210                 215                 220

Phe Met Thr Lys Asp Glu Asp Leu Tyr Asp Lys Thr Ile Glu Asp Val
225                 230                 235                 240

Phe Asp Asp Glu Val Phe Asp Ser Thr Phe Trp Leu Tyr Trp Arg Thr
                245                 250                 255

Met Phe Ala Phe Glu Asn Trp His Ser Ala Leu Glu Met Lys Leu Tyr
            260                 265                 270

Phe Gln Arg Phe Ile His His Ile Ser Gly Leu Pro Asp Phe Ser Ala
        275                 280                 285

Leu Lys Phe Thr Lys Tyr Asn Gln Tyr Glu Ser Leu Ile Leu Pro Met
    290                 295                 300

Val Lys Tyr Leu Glu Ala Ala Gly Val Thr Phe Gln Tyr Asn Thr Glu
305                 310                 315                 320

Val Thr Asn Val Ile Phe Asp Met Arg Asp Gly Lys Lys Ile Ala Lys
                325                 330                 335

Thr Ile Glu Cys Arg Val Asn Gly Thr Glu Lys Gly Ile Pro Leu Thr
            340                 345                 350

Glu Asn Asp Leu Val Phe Val Thr Asn Gly Ser Cys Thr Glu Gly Thr
        355                 360                 365

Ile Tyr Gly Asp Gln Asn His Ala Pro Asn Gly Asp Ala Glu Val Arg
    370                 375                 380

Thr Ser Gly Cys Trp Ser Leu Trp Lys Asn Ile Ala Ala Gln Asp Pro
385                 390                 395                 400

Ser Phe Gly His Pro Glu Lys Phe Cys Ser Asp Ile Ser Lys Thr Asn
                405                 410                 415

Trp Glu Ser Ala Thr Val Thr Thr Leu Asp Asp Lys Ile Ile Pro Tyr
            420                 425                 430

Ile Val Asp Ile Cys Lys Arg Asp Pro Arg Thr Gly Lys Val Val Thr
        435                 440                 445

Gly Gly Ile Val Ser Cys Gln Asp Ser Ser Trp Leu Leu Ser Trp Thr
    450                 455                 460

Ile Asn Arg Gln Gly Gln Phe Lys Thr Gln Asp Lys Asp Lys Val Cys
465                 470                 475                 480

Val Trp Val Tyr Gly Leu Phe Thr Asp Val Pro Gly Asp Tyr Ile Lys
                485                 490                 495

Lys Pro Met Lys Asp Cys Thr Gly Lys Glu Ile Thr Glu Glu Trp Leu
            500                 505                 510

Tyr His Leu Gly Val Pro Glu Asp Glu Ile Glu Glu Leu Ala Thr Asn
        515                 520                 525

Ser Ala Val Cys Val Pro Thr Met Met Pro Tyr Ile Thr Ala Phe Phe
    530                 535                 540

Met Pro Arg Ala Lys Gly Asp Arg Pro Asp Val Ile Pro Asp Gly Cys
545                 550                 555                 560

Thr Asn Phe Ala Phe Leu Gly Gln Phe Ala Asp Thr Pro Arg Asp Thr
                565                 570                 575

Val Phe Thr Thr Glu Tyr Ser Val Arg Thr Ala Met Glu Ala Val Tyr
            580                 585                 590

Gly Leu Leu Gly Val Asp Arg Gly Val Pro Glu Val Trp Gly Ser Val
        595                 600                 605

Tyr Asp Ile Arg Glu Leu Leu Asp Ser Ser Val Lys Leu Met Asp Gly
    610                 615                 620

Lys Ser Pro Leu Glu Ile Asn Leu Gly Pro Leu Asn Lys Leu Lys Arg
625                 630                 635                 640

Pro Ile Leu Lys Lys Ile Gln Gly Thr Val Ile Glu Lys Val Leu Arg
                645                 650                 655

Asp His Asp Ile Ile Lys Glu Gly Met Leu
            660                 665

<210>  6
<211>  386
<212>  PRT
<213>  Gluconobacter oxydans

<400>  6

Met Arg Asp Lys Gly Val Lys Ile Gln Phe Asn Thr Arg Val His Asp
1               5                   10                  15

Val Asp Leu Gln Ile Arg Gly Asp Glu Lys Thr Val Thr Gly Leu Arg
            20                  25                  30

Ala Met Val Asp Gly Gln Glu Thr Thr Ile Pro Val His Pro Asn Asp
        35                  40                  45

Leu Val Phe Ala Leu Thr Gly Ser Met Thr Glu Gly Thr Ala Tyr Gly
    50                  55                  60

Asp Met Asp Thr Val Pro Val Leu Glu Arg Gly Leu His Glu Pro Gly
65                  70                  75                  80

Glu Glu Ser Asp Trp Thr Leu Trp Arg Asn Leu Ala Arg Lys Ser Pro
                85                  90                  95

Val Phe Gly Arg Pro Asp Lys Phe Tyr Gly Asp Val Lys Gly Ser Met
            100                 105                 110

Trp Glu Ser Ala Thr Leu Thr Cys Arg Pro Ser Pro Phe Val Asp Lys
        115                 120                 125

Leu Lys Glu Leu Ser Val Asn Asp Pro Tyr Ser Gly Lys Thr Val Thr
    130                 135                 140

Gly Gly Val Ile Thr Phe Thr Asp Ser Asn Trp Val Leu Ser Phe Thr
145                 150                 155                 160

Cys Asn Arg Gln Pro His Phe Pro Thr Gln Pro Asp Asp Val Leu Val
                165                 170                 175

Ile Trp Val Tyr Ala Leu Leu Met Asp Lys Glu Gly Asn Tyr Val Lys
            180                 185                 190

Lys Pro Met Pro Ala Cys Thr Gly Arg Glu Ile Leu Ala Glu Leu Cys
        195                 200                 205

Tyr His Leu Gly Ile Val Glu Gln Leu Asp Ala Ile Ala Leu Asn Thr
    210                 215                 220

Lys Val Arg Leu Ala Leu Met Pro Tyr Ile Thr Ala Gln Phe Met Pro
225                 230                 235                 240

Arg Ala Ala Gly Asp Arg Pro His Val Val Pro Glu Gly Cys Thr Asn
                245                 250                 255

Leu Gly Leu Leu Gly Gln Phe Val Glu Thr Ser Asn Asp Ile Ile Phe
            260                 265                 270

Thr Met Glu Ser Ser Val Arg Thr Ala Arg Ile Ala Val Tyr Thr Leu
        275                 280                 285

Leu Lys Leu Pro Lys Gln Val Pro Asp Ile Ser Pro Thr Gln Tyr Asp
    290                 295                 300

Ile Arg Thr Ile Phe Lys Ala Ala Arg Ala Ile Asn Asn Gly Glu Pro
305                 310                 315                 320

Phe Pro Gly Glu Arg Leu Leu His Arg Leu Leu Asp Lys Thr Tyr Phe
                325                 330                 335

Ser His Ile Leu Pro Thr Leu Pro Lys Lys Glu Glu Ala Asp Gly Asp
            340                 345                 350

Asp Phe Gly Asn Asp Leu Ser Gly Leu Phe Gly Lys Gly Ser Glu Ala
        355                 360                 365

Leu His Lys Val Thr Asp Trp Leu Glu Lys Phe Arg Ala Asn Leu Gly
    370                 375                 380

Trp Lys
385

<210>  7
<211>  525
<212>  PRT
<213>  Thermoanaerobacterium thermosaccharolyticum DSM 571

<400>  7

Met Arg Asp Tyr Lys Asn Thr His Ala Tyr Phe Val Gly Gly Gly Ile
1               5                   10                  15

Ala Ser Leu Ala Gly Ala Cys Tyr Leu Ile Arg Asp Cys Ser Phe Pro
            20                  25                  30

Gly Asp His Ile His Ile Phe Glu Glu Leu Asn Val Leu Gly Gly Ser
        35                  40                  45

Asn Asp Gly Ala Gly Asn Pro Glu Thr Gly Tyr Val Ile Arg Gly Gly
    50                  55                  60

Arg Met Leu Asn Asp Glu Thr Tyr Glu Asn Thr Trp Glu Leu Leu Leu
65                  70                  75                  80

Ser Ile Pro Ser Ile Asp Asn Pro Glu Lys Ser Val Arg Glu Glu Ile
                85                  90                  95

Ile Glu Phe Asp Thr Gln His Pro Thr His Ser Asn Ala Arg Leu Val
            100                 105                 110

Asn Asn Asn Gly Glu Val Val Asp Val Ser Ser Met Gly Phe Asp Asn
        115                 120                 125

Asp Asp Arg Ile Ala Leu Ala Lys Leu Ile Val Thr Pro Glu Glu Lys
    130                 135                 140

Leu Asn Met Leu Lys Ile Ser Asp Trp Phe Lys Pro His Phe Phe Glu
145                 150                 155                 160

Thr Asn Phe Trp Tyr Met Trp Ala Thr Thr Phe Ala Phe Gln Pro Trp
                165                 170                 175

His Ser Leu Ala Glu Leu Arg Arg Tyr Met Ile Arg Phe Met His Glu
            180                 185                 190

Phe Pro Arg Ile His Thr Leu Glu Gly Val Thr Arg Thr Pro Tyr Asn
        195                 200                 205

Gln Tyr Asp Ser Leu Ile Leu Pro Ile Lys Lys Phe Leu Glu Asn His
    210                 215                 220

Asn Val Asp Phe Lys Leu Lys Cys Ala Val Thr Asp Leu Asp Phe Lys
225                 230                 235                 240

Asp Thr Asp Lys Ile Thr Val Thr Arg Ile Tyr Tyr Lys Glu Glu Gly
                245                 250                 255

Met Glu Lys Thr Ile Glu Val Asn Glu Ser Asp Val Val Phe Val Thr
            260                 265                 270

Asn Gly Ser Met Thr Glu Gly Tyr Ser Leu Gly Ser Met Thr Lys Pro
        275                 280                 285

Pro Val Leu Asn Asp Lys Gly Ala Ser Trp Lys Leu Trp Asp Lys Ile
    290                 295                 300

Ser Lys Lys Lys Gln Gly Leu Gly Asn Pro Thr Val Phe Asp Asp Asn
305                 310                 315                 320

Ile Gln Gly Ser Lys Trp Glu Ser Phe Thr Val Thr Cys His Asn Ser
                325                 330                 335

Lys Phe Phe Asp Leu Met Glu Lys Phe Ser Arg Asn Lys Ala Gly Thr
            340                 345                 350

Gly Ala Leu Val Thr Phe Lys Asp Ser Asn Trp Phe Met Ser Ile Val
        355                 360                 365

Leu Ala His Gln Pro His Phe Arg Asn Gln Pro Asp Asn Val Lys Val
    370                 375                 380

Phe Trp Gly Tyr Gly Leu Phe Pro Asp Asn Pro Gly Asn Tyr Val Lys
385                 390                 395                 400

Lys Lys Met Ser Glu Cys Val Gly Glu Glu Ile Leu Ile Glu Leu Leu
                405                 410                 415

His His Leu Lys Phe Glu Lys Glu Met Asp Asp Ile Ile Lys Ser Ala
            420                 425                 430

Asn Cys Ile Pro Cys Met Met Pro Phe Ile Thr Ala Gln Phe Met Pro
        435                 440                 445

Arg Ser Ile Gly Asp Arg Pro Gln Val Val Pro Glu Gly Ser Thr Asn
    450                 455                 460

Leu Ala Phe Ile Gly Gln Phe Cys Glu Ile Pro Asp Asp Val Val Phe
465                 470                 475                 480

Thr Glu Glu Tyr Ser Val Arg Ser Ala Arg Ile Ala Val Tyr Lys Leu
                485                 490                 495

Phe Gly Ile Thr Arg Pro Val Glu Pro Ile Asn Gln Tyr Gln Tyr Asp

500                 505                 510

Val Arg Thr Leu Phe Lys Ser Phe Val Thr Met Phe Lys
        515                 520                 525

<210>  8
<211>  554
<212>  PRT
<213>  Alkaliphilus metalliredigens (strain QYMF)

<400>  8

Met Cys Leu Asn Arg Ile Val Tyr Asn Gly Val Thr Arg Lys Asn Leu
1               5                   10                  15

Val Asn Asn Ile Leu Leu Glu Gly Ile Thr Met Lys Lys Asp Tyr Gly
            20                  25                  30

Asn Lys Gln Val Tyr Phe Val Gly Gly Gly Ile Ala Ser Leu Ala Gly
        35                  40                  45

Ala Ala Tyr Leu Ile Arg Asp Cys His Phe Lys Gly Glu Asn Ile His
    50                  55                  60

Ile Leu Glu Gly Met His Ile Leu Gly Gly Ser Asn Asp Gly Met Gly
65                  70                  75                  80

Thr Pro Glu Asp Gly Phe Val Cys Arg Gly Gly Arg Met Leu Asn Glu
                85                  90                  95

Glu Thr Tyr Glu Asn Phe Trp Glu Leu Phe Ser Ser Ile Pro Ser Ile
            100                 105                 110

Glu His Glu Gly Met Ser Val Thr Asp Glu Ile Leu Ala Phe Asp His
        115                 120                 125

Ala His Pro Thr His Ala Asn Ala Arg Leu Ile Asn Lys Asp Gly Glu
    130                 135                 140

Val Leu Asp Val Met Ser Met Gly Phe Asp Lys Glu Asp Gln Met Ala
145                 150                 155                 160

Met Gly Lys Leu Leu Leu Thr Pro Glu Glu Lys Leu Asp His Leu Lys
                165                 170                 175

Ile Ser Asp Trp Phe Gly Pro His Phe Phe Glu Thr Asn Phe Trp Tyr
            180                 185                 190

Met Trp Gln Thr Thr Phe Ala Phe Gln Lys Trp Ser Ser Leu Phe Glu

195                 200                 205

Phe Gln Arg Tyr Met Phe Arg Met Ile Phe Glu Phe Ser Arg Ile Gln
    210                 215                 220

Thr Leu Glu Gly Val Thr Arg Thr Pro Tyr Asn Gln Tyr Glu Ser Ile
225                 230                 235                 240

Ile Leu Pro Met Lys Ala Tyr Leu Asp Asp His Gly Val Asp Phe Thr
                245                 250                 255

Ile Lys Cys Thr Val Thr Asp Leu Asp Phe Lys Glu Gly Glu Gly Ile
            260                 265                 270

Thr Val Thr Thr Ile His Tyr Gln Asp Gln Asp Gly Asp Gly Ile Ile
        275                 280                 285

Arg Leu Gln Glu Asp Asp Ile Cys Ile Val Thr Asn Gly Cys Met Thr
    290                 295                 300

Asp Gly Ala Thr Leu Gly Asp Leu Tyr Thr Pro Ala Pro Leu Lys Pro
305                 310                 315                 320

Glu His Pro Ile Ser Gly Asp Leu Trp Ala Asn Ile Ala Ala Lys Lys
                325                 330                 335

Ser Gly Leu Gly Asp Pro Lys Pro Phe Phe Gly Asn Ala Glu Glu Thr
            340                 345                 350

Asn Trp Glu Ser Phe Thr Val Thr Met Lys Gly Asn Lys Leu Leu Lys
        355                 360                 365

Met Ile Glu Lys Tyr Ser Arg Asn Val Pro Gly Ser Gly Ala Leu Met
    370                 375                 380

Thr Phe Lys Asp Ser Ser Trp Leu Met Ser Met Val Val Ala Ala Gln
385                 390                 395                 400

Pro His Phe Lys Ala Gln Thr Ala Asp Glu Thr Ile Phe Trp Gly Tyr
                405                 410                 415

Ala Leu His Thr Asn Lys Val Gly Asp Tyr Val Lys Lys Pro Met Arg
            420                 425                 430

Asp Cys Thr Gly Glu Glu Met Leu Ile Glu Leu Leu His His Leu His
        435                 440                 445

Phe Asp Glu Glu Met Asp Glu Ile Met Lys Asp Val Val Asn Val Ile
    450                 455                 460

Pro Cys Met Met Pro Tyr Ile Ile Ser Gln Phe Gln Pro Arg Ala Met
465                 470                 475                 480

Thr Asp Arg Pro Gln Val Val Pro Glu Gly Ser Thr Asn Leu Ala Met
                485                 490                 495

Ile Ser Gln Phe Val Glu Ile Pro Glu Asp Met Val Phe Thr Glu Glu
            500                 505                 510

Phe Ser Val Arg Ala Ala Arg Ile Ala Val Tyr Thr Leu Met Gly Leu
        515                 520                 525

Asp Lys Lys Ile Cys Pro Val Thr Pro His Arg Tyr Asp Val Arg Thr
    530                 535                 540

Leu Leu Lys Ala Leu Asn Thr Cys Tyr Arg
545                 550

<210>  9
<211>  563
<212>  PRT
<213>  Mollicutes bacterium D7

<400>  9

Met Lys Thr Lys Thr Lys Leu Leu Thr Gly Ala Ala Leu Leu Ala Gly
1               5                   10                  15

Ala Gly Ala Val Ala Ala Lys Lys Tyr Ala Asp Asn Lys Asp Thr Val
            20                  25                  30

Arg Lys Pro Val Glu Ile Thr Asn Gln Gln Phe Tyr Leu Ile Gly Gly
        35                  40                  45

Gly Leu Ala Ser Met Ala Ala Ala Ala Tyr Leu Ile Gln Asp Gly His
    50                  55                  60

Val Asp Gly Lys Asn Ile His Ile Phe Glu Gly Met Lys Ile Leu Gly
65                  70                  75                  80

Gly Ser Asn Asp Gly Ala Gly Ser Ile Lys Asp Gly Phe Val Cys Arg
                85                  90                  95

Gly Gly Arg Met Leu Asn Glu Glu Thr Tyr Glu Asn Phe Trp Glu Leu
            100                 105                 110

Phe Asp Arg Ile Pro Ser Leu Asp His Pro Gly Gln Ser Val Thr Lys
        115                 120                 125

Glu Ile Leu Asp Phe Asp His Leu His Pro Thr Glu Ala Arg Ala Arg
    130                 135                 140

Leu Ile Asp Arg His Gly Lys Ile Leu Asp Val Lys Ser Met Gly Phe
145                 150                 155                 160

Asp Asn Asn Asp Arg Leu Thr Leu Gly Lys Leu Met Ile Thr Pro Glu
                165                 170                 175

Ser Lys Leu Asp Asp Ile Thr Ile Glu Gln Trp Phe Lys Asp Ala Pro
            180                 185                 190

His Phe Phe Thr Thr Asn Phe Trp Tyr Met Trp Gln Thr Thr Phe Ala
        195                 200                 205

Phe Gln Lys Trp Ser Ser Val Phe Glu Leu Lys Arg Tyr Met Asn Arg
    210                 215                 220

Met Ile Phe Glu Phe Pro Arg Ile Glu Thr Leu Ala Gly Val Thr Arg
225                 230                 235                 240

Thr Pro Tyr Asn Gln Phe Glu Ser Val Ile Leu Pro Ile Lys Lys Tyr
                245                 250                 255

Leu Asp Ser His His Val Asn Phe Val Thr Asn Thr Thr Val Thr Asp
            260                 265                 270

Ile Asp Phe Lys Asp Asp Asp Thr Ile Thr Val Lys Ala Leu Tyr Leu
        275                 280                 285

Asn Lys Asp Gly Lys Asp Glu Lys Ile Ile Leu Asn Asp Asn Asp Ile
    290                 295                 300

Cys Ile Met Thr Asn Ala Cys Met Thr Asp Ser Ala Thr Leu Gly Asp
305                 310                 315                 320

Tyr Lys Thr Pro Ala Pro Lys Pro Val Glu Lys Pro Ile Ser Gly Glu
                325                 330                 335

Leu Trp Tyr Lys Val Ala Gln Lys Lys Pro Asn Leu Gly Asn Pro Glu
            340                 345                 350

Pro Phe Phe Gly Asn Ile Lys Glu Thr Asn Trp Glu Ser Ile Thr Val
        355                 360                 365

Thr Phe Lys Gly Asn Lys Phe Leu Lys Ile Ile Glu Glu Phe Ser Thr
    370                 375                 380

Asn Ile Pro Gly Ser Gly Ala Leu Met Thr Phe Lys Asp Ser Ser Trp
385                 390                 395                 400

Leu Met Ser Met Val Val Ala Ala Gln Pro His Phe Lys Ala Gln Asp
                405                 410                 415

Ala Asn Thr Thr Ile Phe Trp Ala Tyr Gly Leu Tyr Thr Asp Arg Leu
            420                 425                 430

Gly Asp Tyr Ile Lys Lys Pro Met Lys Asp Cys Thr Gly Glu Glu Ile
        435                 440                 445

Phe Asp Glu Leu Leu Tyr His Leu His Leu Ile Asp Arg Lys Glu Glu
    450                 455                 460

Ile Lys Lys Asp Ile Ile Asn Val Ile Pro Cys Met Met Pro Tyr Val
465                 470                 475                 480

Asp Ala Gln Phe Gln Pro Arg Lys Met Ser Asp Arg Pro His Val Val
                485                 490                 495

Pro Lys Gly Ser Thr Asn Phe Ala Met Ile Ser Gln Phe Val Glu Ile
            500                 505                 510

Pro Glu Asp Met Val Phe Thr Glu Glu Tyr Ser Val Arg Ala Ala Arg
        515                 520                 525

Ile Ala Val Tyr Thr Leu Leu Gly Ile Asn Lys Pro Ile Cys Lys Val
    530                 535                 540

Thr Pro Tyr Asn Lys Asp Pro Lys Val Leu Lys Lys Ala Leu Glu Thr
545                 550                 555                 560

Ala Tyr Arg

<210>  10
<211>  529
<212>  PRT
<213>  Haloarcula marismortui

<400>  10

Met Thr Ile His Gln His Pro His Val Thr Asp Arg Asp Ala His Phe
1               5                   10                  15

Val Gly Gly Gly Ile Ala Ser Leu Ala Gly Ala Ala Phe Leu Val Arg
            20                  25                  30

Asp Gly Asn Met Pro Gly Glu Asn Ile His Val Tyr Glu Lys Leu Asp
        35                  40                  45

Val Val Gly Gly Ala Met Asp Gly Ala Gly Asp Pro Asp Glu Ala Tyr
    50                  55                  60

Leu Ile Arg Gly Gly Arg Met Phe Asn Tyr Pro Ala Tyr Glu Cys Thr
65                  70                  75                  80

Trp Asp Leu Phe Glu Thr Ile Pro Ser Leu Glu Asp Asp Ser Arg Ser
                85                  90                  95

Val Lys Ala Val Met Asp Glu Phe Asn Glu Thr His Pro Ser Tyr Ala
            100                 105                 110

Lys Thr Arg Leu Met His Glu Gly Glu Arg Ile Asp Ala Gly Glu Tyr
        115                 120                 125

Gly Leu Thr Arg Gln His Arg Gln Ser Ile Ala Arg Leu Leu Leu Thr
    130                 135                 140

Pro Glu Pro Arg Leu Gly Asp Thr Arg Ile Glu Glu Trp Phe Asp Glu
145                 150                 155                 160

Ser Phe Leu Glu Thr Asn Phe Trp Tyr Val Trp Ala Thr Ile Phe Ala
                165                 170                 175

Phe Gln Pro Trp His Ser Val Ala Glu Val Arg Arg Tyr Met Tyr Arg
            180                 185                 190

Phe Leu His Glu Phe Pro Gln Leu His Thr Met Glu Gly Ile Asp Arg
        195                 200                 205

Thr Lys Tyr Asn Gln Tyr Asp Ser Met Ile Leu Pro Leu Arg Arg Trp
    210                 215                 220

Leu Glu Asp Arg Gly Val Ser Phe Glu Tyr Gly His Glu Val Thr Asp
225                 230                 235                 240

Met Asp Ile Val Pro Ser Arg Thr Gly Arg Thr Val Glu Arg Leu Tyr
                245                 250                 255

Cys Glu Thr Asp Ser Gly Thr Glu Thr Val Pro Val Glu Pro Ser Asp
            260                 265                 270

Leu Val Phe Val Thr Asn Gly Ser Met Thr Asp Gly Ser Ser Ile Gly
        275                 280                 285

Gly Met Asp Glu Ala Pro Glu Leu Asn Glu Thr Gly Ala Ser Trp Glu
    290                 295                 300

Leu Trp Lys Ser Ile Ala Glu Asp Asn Pro Gln Phe Gly Asn Pro Ser
305                 310                 315                 320

Val Phe Ala Asp Asn Val Thr Glu Thr Lys Trp Glu Ser Phe Thr Val
                325                 330                 335

Thr Leu His Asn Thr Asp Leu Phe Asp His Ile Val Asp Phe Thr Asn
            340                 345                 350

Glu Glu Pro Gly Asn Gly Leu Val Thr Tyr Leu Gly Ser Asn Trp Leu
        355                 360                 365

Leu Ser Thr Val Val Ala Ala Gln Pro His Phe Ala Asn Gln Pro Asp
    370                 375                 380

Asp Val Lys Val Phe Trp Gly Tyr Ala Leu Phe Pro Asp Arg Glu Gly
385                 390                 395                 400

Asn Tyr Val Glu Lys Pro Met Ser Glu Cys Thr Gly Arg Glu Ile Leu
                405                 410                 415

Gln Glu Leu Cys Tyr His Leu Gln Cys Glu Glu Arg Leu Pro Glu Ile
            420                 425                 430

Leu Asp Asp Val Thr Cys Ile Pro Ala Met Met Pro Phe Ile Thr Ala
        435                 440                 445

His Phe Gln Pro Arg Glu Pro Gly Asp Arg Pro Asp Val Val Pro Asp
    450                 455                 460

Gly Ser Asn Asn Leu Ala Phe Leu Gly Gln Tyr Ala Glu Gln Pro Arg
465                 470                 475                 480

Asp Val Val Phe Thr Val Glu Tyr Ser Val Arg Ser Ala Met Thr Ala
                485                 490                 495

Val Tyr Glu Met Leu Asp Leu Asp Arg Glu Val Pro Pro Val Ser Lys
            500                 505                 510

His Tyr Arg Lys Pro Gly Val Ile Ala Asp Thr Val Arg Ala Ala Tyr

515                 520                 525

Arg

<210>  11
<211>  585
<212>  PRT
<213>  Nectria haematococca (strain 77-13-4 / FGSC 9596 / MPVI)

<400>  11

Met Tyr Tyr Ser Lys Gly Asn Tyr Glu Ala Phe Ala Arg Pro Leu Lys
1               5                   10                  15

Pro Glu Gly Ile Glu Lys Lys Lys Ala Trp Ile Val Gly Ser Gly Leu
            20                  25                  30

Ser Gly Leu Ser Thr Ala Ala Phe Leu Val Arg Asp Ala Gln Met Pro
        35                  40                  45

Gly Lys Asn Ile Thr Ile Leu Glu Glu Leu Asn Ile Ser Gly Gly Ala
    50                  55                  60

Leu Asp Gly Ile His Asp Ser Glu Lys Gly Phe Val Met Arg Gly Gly
65                  70                  75                  80

Arg Glu Met Glu Ser His Phe Glu Cys Leu Trp Asp Leu Phe Arg Ser
                85                  90                  95

Val Pro Ser Leu Glu Glu Asn Glu Ala Ser Val Leu Asp Glu Phe Tyr
            100                 105                 110

Trp Leu Asn Lys Arg Asp Pro Asn Phe Ser Leu Gln Arg Ala Thr Ile
        115                 120                 125

Lys Gln Gly Gln Asp Ala Glu Thr Gly Thr Leu Phe Thr Leu Ser Glu
    130                 135                 140

Lys Ala Gln Ala Glu Val Thr Lys Leu Phe Leu Ala Thr Arg Lys Glu
145                 150                 155                 160

Val Glu Asn Val Arg Ile Asp Gln Val Phe Ser Gln Glu Phe Phe Asp
                165                 170                 175

Ser Asn Phe Trp Met Tyr Trp Gln Thr Met Phe Ala Phe Gln Thr Trp
            180                 185                 190

His Ser Ala Leu Glu Met Lys Leu Tyr Leu His Arg Phe Val Asn His

195                 200                 205

Ile Arg Gly Met Pro Asp Phe Ser Thr Leu Lys Phe Thr Arg Tyr Asn
    210                 215                 220

Gln Tyr Glu Ser Leu Val Val Pro Leu His Thr Trp Leu Glu Glu Lys
225                 230                 235                 240

Gly Val Val Phe Gln Tyr Ser Thr Glu Val Gln Asp Val Asp Phe Asp
                245                 250                 255

Ile Lys Glu Asp Lys Lys Thr Ala Thr Tyr Ile His Trp Ile Lys Asp
            260                 265                 270

Gly Lys Lys Ser Gly Gln Ala Leu Ser Pro Asp Asp Leu Val Phe Met
        275                 280                 285

Thr Ile Gly Ser Leu Thr Glu Asn Thr Gly Tyr Gly Asp His His Thr
    290                 295                 300

Val Pro Lys Leu Leu Glu Gly Pro Ala Pro Ala Trp Asp Leu Trp Arg
305                 310                 315                 320

Arg Ile Ala Ala Lys Asp Pro Ala Phe Gly Arg Pro Glu Val Phe Cys
                325                 330                 335

Gly Asp Ile Lys Ala Thr Lys Trp Glu Ser Ala Thr Phe Gln Thr Leu
            340                 345                 350

Asp Ala Arg Ile Pro Glu Tyr Ile Gln Lys Val Cys Lys Arg Asp Pro
        355                 360                 365

Leu Ser Gly Lys Val Val Thr Gly Gly Ile Val Thr Val Arg Asp Ser
    370                 375                 380

Ser Trp Ile Met Ser Trp Thr Val Asn Arg Gln Pro His Phe Lys Asn
385                 390                 395                 400

Gln Pro Lys Gly Ser Val Val Gly Trp Ile Tyr Gly Leu Leu Val Glu
                405                 410                 415

Lys Pro Gly Asp Tyr Val Lys Lys Pro Met Gln Glu Cys Thr Gly Glu
            420                 425                 430

Glu Ile Val Gln Glu Trp Leu Tyr His Leu Gly Val Pro Thr Ser Glu
        435                 440                 445

Ile Ala Thr Leu Ala Ala Asp Ala Val Lys Cys Val Pro Thr Met Met
    450                 455                 460

Pro Tyr Ile Thr Ala Phe Phe Met Pro Arg Gln Ala Gly Asp Arg Pro
465                 470                 475                 480

Asp Val Val Pro Ala Gly Ser Gln Asn Phe Ala Phe Ile Gly Gln Phe
                485                 490                 495

Ala Glu Thr Thr Arg Asp Cys Ile Phe Thr Thr Glu Tyr Ala Val Arg
            500                 505                 510

Thr Gly Met Glu Ser Val Tyr Gln Leu Thr Gly Val Asp Arg Ala Val
        515                 520                 525

Pro Glu Val Phe Gly Ser Thr Tyr Asp Val Arg Val Leu Leu Asp Ala
    530                 535                 540

Met Ser Gln Leu Arg Asp Gly Lys Glu Leu Ala Pro Trp Leu Pro Glu
545                 550                 555                 560

Arg Ile Arg Ala Phe Leu Met Ser Lys Leu Gln Gly Ser Gln Ile Gly
                565                 570                 575

Gln Met Leu His Glu Tyr His Leu Val
            580                 585